# EUROPÄISCHE PATENTSCHRIFT

(11) **EP 2 236 109 B1**
(45) Veröffentlichungstag und Bekanntmachung des Hinweises auf die Patenterteilung: **22.10.2014**
(21) Anmeldenummer: 09004892.7
(22) Anmeldetag: 02.04.2009
(51) Int. Cl.: A61F 9/01, A61B 19/00, A61F 9/008, A61F 9/009

(54) **System zum Definieren von Schnitten in Augengewebe**
System for defining cuts in eye tissue
Système destiné à la définition de découpes dans des tissus oculaires

(43) Veröffentlichungstag der Anmeldung: 06.10.2010
(73) Patentinhaber: SIE AG, Surgical Instrument Engineering, 2562 Port (CH)
(72) Erfinder: Rathjen, Christian, 28197 Bremen (DE)
(74) Vertreter: Rentsch Partner AG

(56) Entgegenhaltungen:
- DE-A1- 4 304 571
- US-A1- 2008 319 428
- US-E1- R E37 585

## Beschreibung

### Technisches Gebiet

Die vorliegende Erfindung betrifft ein System zum Definieren von Schnitten in Augengewebe. Die vorliegende Erfindung betrifft insbesondere ein computergestütztes System zum Definieren von einem oder mehreren mittels Femtosekundenlaserpulsen in einem menschlichen Auge auszuführenden Gewebeschnitten.

### Stand der Technik

Die Lasertechnologie wird seit Jahrzehnten für den universellen Einsatz zum Schneiden von unterschiedlichsten Materialien propagiert. Im Bereich der Ophthalmologie hat der Laser allerdings für viele Anwendungen noch nicht das Skalpell zum Schneiden von Augengewebe ersetzt. Die bekannten Lasersysteme ermöglichen nämlich einem Benutzer nicht, im Augengewebe weitgehend beliebige Schnitte zu erzeugen, wie z.B. in WO9425107 oder WO9409849 beschrieben wird und wie beispielsweise mit einem Skalpell möglich ist. Auch wenn ein Femtosekundenlaser überdies ermöglicht im Augengewebe mittels Femtosekundenlaserpulsen Schnitte durchzuführen, die mit einem Skalpell nicht möglich sind (z.B. einstichfreie Schnitte innerhalb des Gewebes), sind die bekannten ophthalmologischen Lasersysteme hinsichtlich der für den Benutzer verfügbaren Schneideflexibilität stark eingeschränkt. Grund dafür ist insbesondere auch, dass es für ein vom Benutzer gewünschtes Schnittergebnis eine Vielzahl von nicht effektiven und unter Umständen sogar patientenschädlichen Parametrisierungen des Lasersystems gibt. Die bekannten ophthalmologischen Lasersysteme weisen eine für die Benutzer eingeschränkte Schneideflexibilität auf, weil der typische Benutzer kein Laserexperte und im Allgemeinen nicht in der Lage ist, die Strahlablenkung und Pulssteuerung eines Lasers effizient und sicher durchzuführen. Die bekannten ophthalmologischen Lasersysteme ermöglichen in der Regel weder eine Operationsplanung mit einfacher Erkennung respektive Verhinderung schädlicher oder unmöglicher Schnittführungen, noch eine Minimierung der Eingriffszeit oder automatische Erzeugung einer Schrittsequenz Schliesslich ermöglichen die bekannten ophthalmologischen Lasersysteme auch keine Behandlung eines Auges auf der Basis bereits durchgeführter und bewährter Eingriffe, die nicht Bestandteil von Standardprozeduren sind, wie beispielsweise das Schneiden eines Corneaflaps.

In der Offenlegungsschrift DE 43 04 571 wird ein Verfahren zur Planung und Kontrolle eines chirurgischen Eingriffs beschrieben, bei welchen basierend auf Computertomographien oder Röntgenbildern, beispielsweise durch Eingabe von Koordinaten, optimale Schnittflächen bestimmt werden, an denen während des Eingriffs das Gewebe getrennt werden soll. Gemäss DE 43 04 571 wird eine Abbildung der geplanten Schnittflächen erzeugt, die während des Eingriffs einer Ist-Abbildung des Operationsfelds überlagert oder auf das Operationsfeld selbst projiziert wird, um so dem Chirurgen als Leitlinie und Kontrolle beim Vornehmen der Gewebeschnitte mittels eines Skalpells zu dienen.

In der Patentanmeldung US 2008/0319428 wird ein Verfahren und eine Vorrichtung für die refraktive Augenchirurgie beschrieben, welche basierend auf Messdaten und Angaben über das eingeschränkte Sehvermögen ein Volumen innerhalb der Cornea definieren, das entfernt werden soll, um die gewünschte refraktive Korrektur zu erreichen. Gemäss US 2008/0319428 werden basierend auf der Grenzfläche, die das Volumen innerhalb der Cornea abgrenzt, Steuerdaten zur Steuerung eines Lasers erzeugt, welche ein dreidimensionales Muster von Zielpunkten definieren. Die Vorrichtung weist eine Anzeige zur Visualisierung der Steuerdaten auf und ermöglicht dem Chirurgen die Veränderung der Steuerdaten.

### Darstellung der Erfindung

Es ist eine Aufgabe der vorliegenden Erfindung ein computergestütztes System zum Definieren von einem oder mehreren mittels Femtosekundenlaserpulsen in einem menschlichen Auge auszuführenden Gewebeschnitten vorzuschlagen, welches zumindest einige Nachteile der bekannten Systeme nicht aufweist. Es ist insbesondere eine Aufgabe der vorliegenden Erfindung ein computergestütztes System zum Definieren der Gewebeschnitten vorzuschlagen, welches einem Benutzer die durch Femtosekundenlasertechnik ermöglichte Schneideflexibilität verfügbar macht. Es ist insbesondere eine weitere Aufgabe der vorliegenden Erfindung ein computergestütztes System zum Definieren der Gewebeschnitten basierend auf bestehenden Definitionen von bewährten Gewebeschnitten vorzuschlagen.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass ein computergestütztes System bereitgestellt wird zum Generieren eines dreidimensionalen Schnittmusters, welches einen oder mehrere mittels Femtosekundenlaserpulsen in einem menschlichen Auge auszuführende Gewebeschnitte definiert. Das computergestützte System umfasst einen Datenspeicher mit Augendaten, welche ein dreidimensionales Augenmodell definieren, und einen Referenzgenerator, welcher eingerichtet ist, mindestens eine geometrische Referenz mit Bezug zum dreidimensionalen Augenmodell zu definieren und zu speichern. Überdies umfasst das computergestützte System einen Schnittflächeneditor, welcher eingerichtet ist, basierend auf Benutzerinstruktionen mindestens eine Schnittfläche zu definieren und die mindestens eine Schnittfläche mit Bezug zur mindestens einen geometrischen Referenz im dreidimensionalen Augenmodell zu positionieren. Schliesslich umfasst das computergestützte System einen Schnittmustergenerator, welcher eingerichtet ist, das dreidimensionale Schnittmuster zur Definition von Gewebeschnitten basierend auf der mindestens einen oder mehreren im dreidimensionalen Augenmodell positionierten Schnittflächen zu generieren und zu speichern. Die Definition von Gewebeschnitten in einem Auge durch Erzeugung eines dreidimensionalen Schnittmusters, das auf mindestens einer oder mehreren in einem dreidimensionalen Augenmodell durch den Benutzer bestimmten und positionierten Schnittfläche basiert, erlaubt es dem Benutzer die durch Femtosekundenlasertechnik ermöglichte Schneideflexibilität für einen einmaligen oder mehrmaligen Einsatz bei einer respektive mehreren Augenbehandlungen verfügbar zu machen, da die Gewebeschnitte vorbereitend gezielt und effizient im dreidimensionalen Augenmodell definiert werden können und dafür keine Konfigurationen, Manipulationen oder Eingriffe während einer chirurgischen Behandlung direkt am Auge notwendig sind.

In einer Ausführungsvariante ist der Schnittflächeneditor eingerichtet, die mindestens eine Schnittfläche basierend auf einer benutzerdefinierten Schnittlinie und einer benutzerdefinierten Schnittlinientrajektorie zu definieren. Dies ermöglicht es einem Benutzer, Schnittflächen mit unterschiedlichsten Formen flexibel auf der Basis einer Schnittlinie zu definieren, welche bei der Ausführung des Schnitts wie eine Klinge oder eine Drahtschneide entlang der Schnittlinientrajektorie durch das Augengewebe geführt werden.

In einer weiteren Ausführungsvariante ist der Schnittflächeneditor eingerichtet, die mindestens eine Schnittfläche basierend auf mindestens einem Flächenelement zu definieren, welches basierend auf Benutzerinstruktionen aus einer Liste von mehreren unterschiedlichen Flächenelementen auswählbar ist. Dies ermöglicht eine einfache Definition von vordefinierten Schnittformen, die überdies vom Benutzer noch angepasst werden können.

In einer Ausführungsvariante umfasst das computergestützte System einen Applikatorselektor, welcher eingerichtet ist, basierend auf Benutzerinstruktionen ein beim Ausführen der Gewebeschnitte auf das Auge aufzusetzendes Applikationselement zu bestimmen, und ein Deformationsmodul, welches eingerichtet ist, basierend auf den Augendaten und dem bestimmten Applikationselement, ein deformiertes dreidimensionales Augenmodell zu bestimmen, welches das Auge im Zustand des aufgesetzten Applikationselements repräsentiert. In einer Ausführungsvariante ist der Referenzgenerator eingerichtet, die geometrische Referenz mit Bezug zum deformierten dreidimensionalen Augenmodell zu definieren und zu speichern; der Schnittflächeneditor ist eingerichtet, die mindestens eine Schnittfläche mit Bezug zur mindestens einen geometrischen Referenz im deformierten dreidimensionalen Augenmodell zu positionieren; und der Schnittmustergenerator ist eingerichtet, das dreidimensionale Schnittmuster basierend auf der mindestens einen oder mehreren im deformierten dreidimensionalen Augenmodell positionierten Schnittflächen zu generieren und zu speichern. Das heisst, Schnittmuster können basierend auf direkt im deformierten Zustand des Augenmodells definierten Schnittflächen erzeugt werden. In einer weiteren Ausführungsvariante ist das Deformationsmodul eingerichtet, basierend auf dem dreidimensionalen Schnittmuster und dem bestimmten Applikationselement ein deformiertes dreidimensionales Schnittmuster zu bestimmen, welches auf einem deformierten Zustand der mindestens einen oder mehreren im dreidimensionalen Augenmodell positionierten Schnittflächen basiert, wobei der deformierte Zustand durch das deformierte dreidimensionale Augenmodell definiert ist. Das heisst, deformierte Schnittmuster können basierend auf Schnittflächen erzeugt werden, die im undeformierten Zustand des Augenmodells definiert wurden. Das Deformationsmodul ermöglicht somit das Augenmodell an den Zustand des Auges bei aufgesetztem Applikationselement anzupassen und bereits im undeformierten Zustand definierte Schnittflächen und/oder geometrische Referenzen in den deformierten Zustand zu transformieren respektive neue oder weitere Schnittflächen und/oder geometrische Referenzen direkt im deformierten Zustand zu definieren.

In einer Ausführungsvariante umfasst das computergestützte System einen Sequenzgenerator, welcher eingerichtet ist, für die Schnittflächen der im dreidimensionalen Schnittmuster definierten Gewebeschnitte eine Ausführungssequenz automatisch oder basierend auf Benutzerinstruktionen zu bestimmen, wobei verhindert wird, dass eine gemäss der Ausführungssequenz früher zu erzeugende Schnittfläche eine fokussierte Einstrahlung der Femtosekundenlaserpulse zur Erzeugung einer gemäss der Ausführungssequenz später zu erzeugenden Schnittfläche verdeckt oder abschattet. In einer weiteren Ausführungsvariante ist der Sequenzgenerator eingerichtet, eine Verdeckung oder Abschattung einer später zu erzeugenden Schnittfläche durch eine früher zu erzeugende Schnittfläche basierend auf einem durch fokussierte Einstrahlung der Femtosekundenlaserpulse definierten Strahlkonus zu bestimmen. Der Sequenzgenerator ermöglicht somit eine Plausibilitätsprüfung der Reihenfolge, in der die Schnittflächen eines Schnittmusters ausgeführt werden, basierend auf Abschattungen und/oder Verdeckungen durch Schnittflächen.

In einer weiteren Ausführungsvariante ist der Sequenzgenerator eingerichtet, die Ausführungssequenz für die Schnittflächen so zu bestimmen, dass eine erwartete thermische Belastung des Auges einen definierten Grenzwert nicht übersteigt. Der Sequenzgenerator verhindert damit unerwünschte Beschädigungen von Augengewebe durch Überbelastungen.

In einer Ausführungsvariante umfasst das computergestützte System einen Maskenselektor, welcher eingerichtet ist, basierend auf Benutzerinstruktionen eine beim Ausführen der Gewebeschnitte vor das Auge zu positionierende Projektionsmaske zu bestimmen, und basierend auf der bestimmten Projektionsmaske und einem durch fokussierte Einstrahlung der Femtosekundenlaserpulse definierten Strahlkonus zu überprüfen, ob die Schnittflächen der im dreidimensionalen Schnittmuster definierten Gewebeschnitte ohne Abschattung durch die Projektionsmaske erzeugbar sind. Der Maskenselektor ermöglicht somit eine Plausibilitätsprüfung des Schnittmusters hinsichtlich einer unerwünschten Abschattung von Schnittflächen durch eine selektierte Projektionsmaske.

In einer weiteren Ausführungsvariante umfasst das computergestützte System ein Visualisierungsmodul, welches eingerichtet ist, basierend auf den Augendaten eine Visualisierung des Auges und einen durch fokussierte Einstrahlung der Femtosekundenlaserpulse definierten Strahlkonus auf einer Anzeige darzustellen; und einen Schnittaufzeichner, welcher eingerichtet ist, basierend auf Benutzerinstruktionen im visualisierten Auge durch Bewegen des Strahlkonus einen virtuellen Gewebeschnitt zu erzeugen und den virtuellen Gewebeschnitt als dreidimensionales Schnittmuster zu speichern. Der Schnittaufzeichner ermöglicht dem Benutzer somit die Definition einer Schnittfläche und/oder eines Schnittmusters durch Ausführen eines virtuellen Gewebeschnitts im visualisierten Augenmodell.

In einer Ausführungsvariante umfasst das computergestützte System einen Schnittsimulator, welcher eingerichtet ist, basierend auf den Augendaten und dem gespeicherten dreidimensionalen Schnittmuster eine Ausführung der durch das dreidimensionale Schnittmuster definierten Gewebeschnitte zu simulieren und auf einer Anzeige zu visualisieren; und einen Schnittmustereditor, welcher eingerichtet ist, basierend auf Benutzerinstruktionen das gespeicherte dreidimensionale Schnittmuster durch eine oder mehrere Operationen aus der folgenden Liste anzupassen: Repositionieren einer Schnittfläche, Reorientieren einer Schnittfläche, Ändern einer Schnittrichtung einer Schnittfläche, Löschen einer Schnittfläche, Löschen eines Schnittflächenteils, Verändern einer Schnittfläche, Hinzufügen einer Schnittfläche, Duplizieren einer Schnittfläche, Ändern einer geometrischen Referenz, Ändern einer Ausführungssequenz für die Schnittflächen, Ändern eines beim Ausführen der Gewebeschnitte auf das Auge aufzusetzenden Applikationselements, Ändern einer beim Ausführen der Gewebeschnitte vor das Auge zu positionierenden Projektionsmaske und Ändern eines durch fokussierte Einstrahlung der Femtosekundenlaserpulse definierten Strahlkonus. Der Schnittsimulator ermöglicht somit die Auswirkungen von Änderungen am dreidimensionalen Schnittmuster durch Simulation zu überprüfen, beispielsweise können Applikationselemente unterschiedlicher Formen auf ihre Wirkung bezüglich einer refraktiven Korrektur überprüft und gestestet werden.

In einer weiteren Ausführungsvariante ist der Schnittmustergenerator eingerichtet, im dreidimensionalen Schnittmuster Gewebeschnitte durch eine oder mehrere in Bezug zu einer geometrischen Referenz positionierte Schnittflächen zu definieren, wobei eine Schnittfläche jeweils durch einen oder mehrere Parameter aus der folgenden Liste definiert ist: Position, Schnittlinie, Schnittlinientrajektorie, Schnittrichtung, Flächenelementklasse, Flächenelementabmessungen, Flächenelementkrümmung und Flächenelementorientierung, und wobei den Schnittflächen eine Ausführungssequenz zugeordnet ist.

In einer Ausführungsvariante ist der Schnittmustergenerator eingerichtet, die Schnittflächen im dreidimensionalen Schnittmuster in Form von Makroinstruktionen zu definieren. Je nach Ausführung der ophthalmologischen Laservorrichtung erfolgt deren Steuerung direkt über die Makroinstruktionen oder basierend auf entsprechenden Pulsrasterinstruktionen, die den Femtosekundenlaser steuern.

In einer weiteren Ausführungsvariante ist der Schnittflächeneditor eingerichtet, basierend auf Benutzerinstruktionen die mindestens eine Schnittfläche durch eine oder mehrere Operationen aus der folgenden Liste anzupassen: Skalieren der Grösse der Schnittfläche, Begrenzen der Schnittfläche entlang einer oder mehreren Grenzlinien, Verändern einer Krümmung der Schnittfläche, Ändern einer Orientierung der Schnittfläche, Ändern einer Schnittrichtung der Schnittfläche und Ändern einer Schnittklasse der Schnittfläche, wobei die Schnittklasse durch ein oder mehrere Elemente aus der folgenden Liste von Schnittklassen definiert ist: Schnitte mit einer als Fläche zweiter Ordnung definierten Schnittfläche, Schnitte mit einer als Splinefläche definierten Schnittfläche, Schnitte mit einer durch Schnittlinie und Schnittlinientrajektorie definierten Schnittfläche und Schnitte mit einer durch ein gespeichertes dreidimensionales Schnittmuster definierten Schnittfläche.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
- Figur 1:: zeigt schematisch ein Computersystem zum Generieren eines dreidimensionalen Schnittmusters, welches mit einer Laservorrichtung zur Ausführung von Gewebeschnitten an einem menschlichen Auge verbunden ist.
- Figur 2:: zeigt ein Blockdiagramm, welches schematisch funktionale Module und Datenspeicher des Computersystems zum Generieren eines dreidimensionalen Schnittmusters der Gewebeschnitte illustriert.
- Figur 3:: zeigt ein Flussdiagramm, welches eine mögliche Sequenz von Schritten zum Generieren des dreidimensionalen Schnittmusters illustriert.
- Figur 4:: zeigt ein Flussdiagramm, welches eine mögliche Sequenz von benutzerselektierten Konfigurations- und Vorbereitungsschritten illustriert.
- Figur 5:: zeigt ein Flussdiagramm, welches eine mögliche Sequenz von Schritten zum Definieren des dreidimensionalen Schnittmusters illustriert.
- Figur 6:: zeigt ein Flussdiagramm, welches eine mögliche Sequenz von Schritten zum Definieren von Schnitten gemäss verschiedenen Schnittklassen illustriert.
- Figur 7:: zeigt ein Flussdiagramm, welches eine mögliche Sequenz von Schritten zum Definieren und Überprüfen einer Ausführungssequenz einzelner Schnitte eines Schnittmusters illustriert.
- Figur 8:: zeigt im Querschnitt schematisch einen Ausschnitt einer Cornea in einem undeformierten und in einem durch Applanation deformierten Zustand.
- Figur 9:: zeigt im Querschnitt schematisch ein Auge mit einem Keratokonus.
- Figur 10:: zeigt in der Aufsicht schematisch ein Auge mit einem Keratokonus.
- Figur 11:: zeigt in der Aufsicht schematisch ein Auge mit verschiedenen möglichen geometrischen Referenzen.
- Figur 12:: zeigt in der Aufsicht schematisch ein Auge mit bestimmten geometrischen Referenzen und einem Schnittmuster zur Stützung des Keratokonus.
- Figur 13:: zeigt in der Aufsicht schematisch eine Cornea mit einem Schnittmuster zur Stützung des Keratokonus mittels einer mechanischen Stützeinlage.
- Figur 14:: zeigt in der Aufsicht schematisch eine Cornea mit einem Schnittmuster zum Glattziehen der durch den Keratokonus erzeugten Ausbeulung der Cornea.
- Figur 15:: zeigt in der Aufsicht schematisch eine Cornea mit einem Schnittmuster zur Stützung des Keratokonus.
- Figur 16:: zeigt in der Aufsicht schematisch eine Cornea mit einem Schnittmuster zur Stützung des Keratokonus mittels zwei sich kreuzenden Stützeinlagen.
- Figur 17:: zeigt in der Aufsicht schematisch eine Cornea mit einem Schnittmuster für eine lokale Keratoplastik.
- Figur 18:: zeigt ein Block- und Datenflussdiagramm, welches schematisch eine Anordnung des Computersystems zum Generieren des Schnittmusters als Teil eines computergestützten Operationsplanungssystems illustriert.
- Figur 19:: zeigt ein Querschnittbild durch ein Corneasegment im undeformierten Zustand, nachdem im deformierten Zustand ein Corneaflap (Cornealappen) geschnitten wurde.

### Wege zur Ausführung der Erfindung

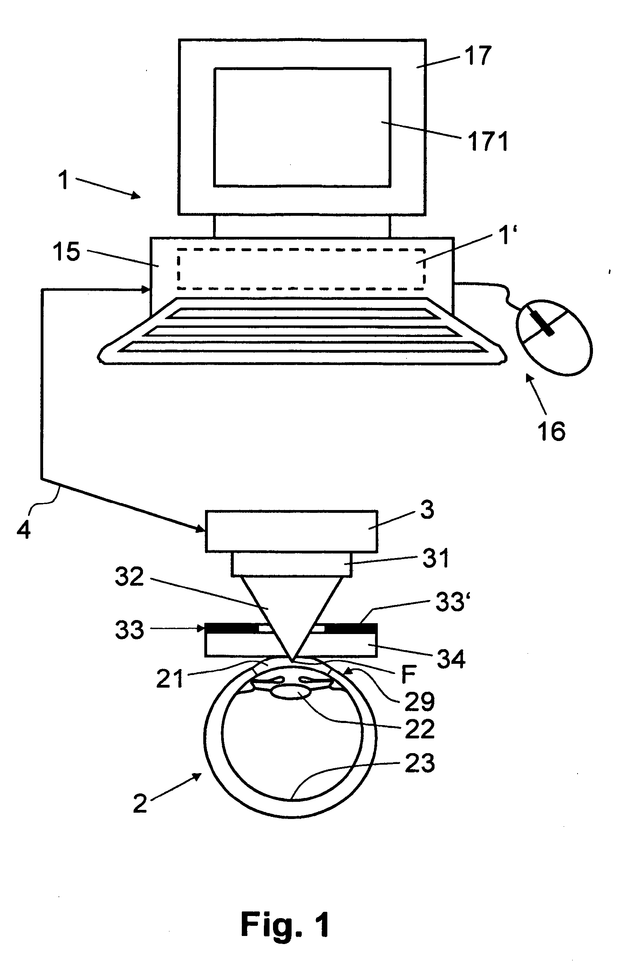

In der Figur 1 bezieht sich das Bezugszeichen 1 auf ein Computersystem zum Generieren eines dreidimensionalen Schnittmusters, das einen oder mehrere zusammenhängende oder getrennte Gewebeschnitte in einem menschlichen Auge 2 definiert, die durch eine ophthalmologische Laservorrichtung 3 mittels Femtosekundenlaserpulsen auszuführen sind.

Die Laservorrichtung 3 umfasst einen Lichtprojektor 31, welcher die Femtosekundenlaserpulse in einem definierten Strahlkonus 32 auf respektive in das Augengewebe fokussiert. Als Schnitt wird dabei jeweils ein Gewebeabbau respektive eine Gewebeauflösung in einem zusammenhängenden Gebiet des Augengewebes verstanden, das typischerweise durch die Femtosekundenlaserpulse an mindestens zwei verschiedenen Fokuspunkten F bewirkt wird. In den bevorzugten Anwendungen umfasst das Augengewebe verschiedene Augenstrukturen, insbesondere die Cornea 21 (Hornhaut), die Sklera 29, die Linse 22 und die Retina 23 (Netzhaut).

Wie in der Figur 1 dargestellt ist, erfolgt die Behandlung typischerweise in einem durch Aufsetzen eines Applikationselements 34 definierten Zustand des Auges 2. Das Applikationselement 34 ist ein konkav, konvex oder plan ausgestalteter Kontaktkörper, der das Auge 2, insbesondere die Cornea 21, in einen gewünschten Sollzustand deformiert, beispielsweise applaniert. Zudem wird je nach Anwendung eine Projektionsmaske 33 vor das Auge 2 positioniert, welche lichtundurchlässige oder lichtdämpfende Maskenbereiche 33' aufweist, mittels derer das Auge 2 vom Strahlkonus 32 abgeschattet und ein Eindringen der Femtosekundenlaserpulse auf respektive in das Augengewebe verhindert respektive gedämpft wird. Befestigungsmittel wie beispielsweise Saugringe zur Fixierung des Applikationselements 34 und/oder der Projektionsmaske 33 sind nicht dargestellt und werden hier nicht weiter beschrieben.

Das Computersystem 1 umfasst einen oder mehrere Computer 15 mit Eingabeelementen 16 und einer Anzeige 17. Die Eingabeelemente 16 umfassen beispielsweise Tasten und Positionierelemente wie Computermaus, Tastfeld (Touchpad) oder Steuerkugel (Trackball). Die Eingabeelemente 16 und die Anzeige 17 können auch in einem berührungsempfindlichen Bildschirm kombiniert sein. Der Computer 15 ist beispielsweise ein betriebsbereiter mobiler oder fest installierter PC mit einem oder mehreren Prozessoren, Daten- und Programmspeicher.

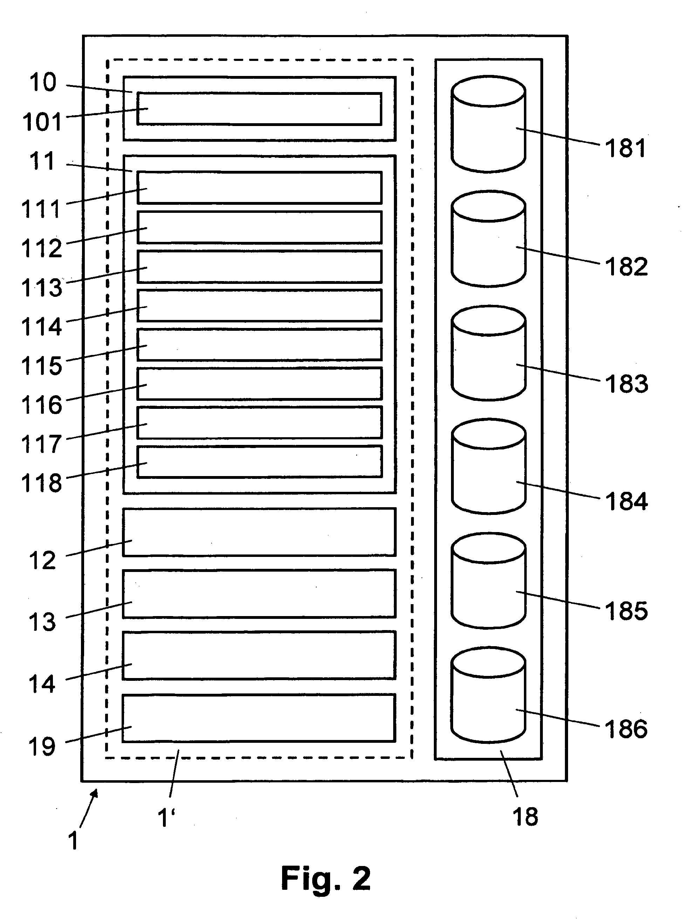

Wie in der Figur 2 schematisch dargestellt ist umfasst das Computersystem 1 mehrere funktionale Module 1', insbesondere ein Steuermodul 10, einen Laserselektor 101, einen Schnittmustereditor 11, ein Deformationsmodul 12, ein Visualisierungsmodul 13, einen Schnittsimulator 14 und ein Kommunikationsmodul 19. Der Schnittmustereditor 11 umfasst einen Maskenselektor 111, einen Applikatorselektor 112, einen Referenzgenerator 113, einen Schnittflächeneditor 114, einen Schnittaufzeichner 115, einen Sequenzgenerator 116 und einen Schnittmustergenerator 117. Die funktionalen Module 1' sind vorzugsweise als programmierte Softwaremodule ausgeführt, die auf einem fest oder entfernbar mit dem Computersystem 1 verbundenen computerlesbaren Datenträger gespeichert sind und Computerprogrammcode zur Steuerung der Prozessoren des Computers 15 umfassen, derart, dass dieser die nachfolgend beschriebenen Funktionen ausführt. Der Fachmann wird verstehen, dass die funktionalen Module 1' in alternativen Ausführungsvarianten teilweise oder vollständig durch Hardwareelemente ausgeführt werden können.

Das Computersystem 1 umfasst zudem einen Datenspeicher 18 mit Datenstrukturen zur Speicherung von Augendaten 181, die ein dreidimensionales Augenmodell definieren, Laserdaten 182, die verschiedene Typen und Konfigurationen von ophthalmologischen Laservorrichtungen 3 definieren, Applikationsdaten 183, die verschiedene Applikationselemente definieren, Maskendaten 184, die verschiedene Projektionsmasken definieren, Flächenelementdaten 185, die verschiedene Flächenelemente definieren, und Schnittmusterdaten 186, die verschiedene dreidimensionale Schnittmuster definieren. Die Datenstrukturen zur Speicherung der Augendaten 181 umfassen zudem optionale Datenstrukturen zur Speicherung von externen Messdaten und Bilddaten, die beispielsweise über einen Kommunikationskanal von externen Mess- respektive Bilderfassungsgeräten eingelesen oder über die Benutzerschnittstelle 171 eingegeben und einem Auge 2 eines Patienten zugeordnet werden. Die Bilddaten umfassen insbesondere auch Trübungen und Einschlüsse, welche visuell überlagert und zur Definition von geometrischen Referenzen verwendet werden.

Das Kommunikationsmodul 19 ist eingerichtet, Daten, insbesondere Schnittmusterdaten, über ein Telekommunikationsnetz, insbesondere über das Internet, mit einem externen computerbasierten Datenserver auszutauschen.

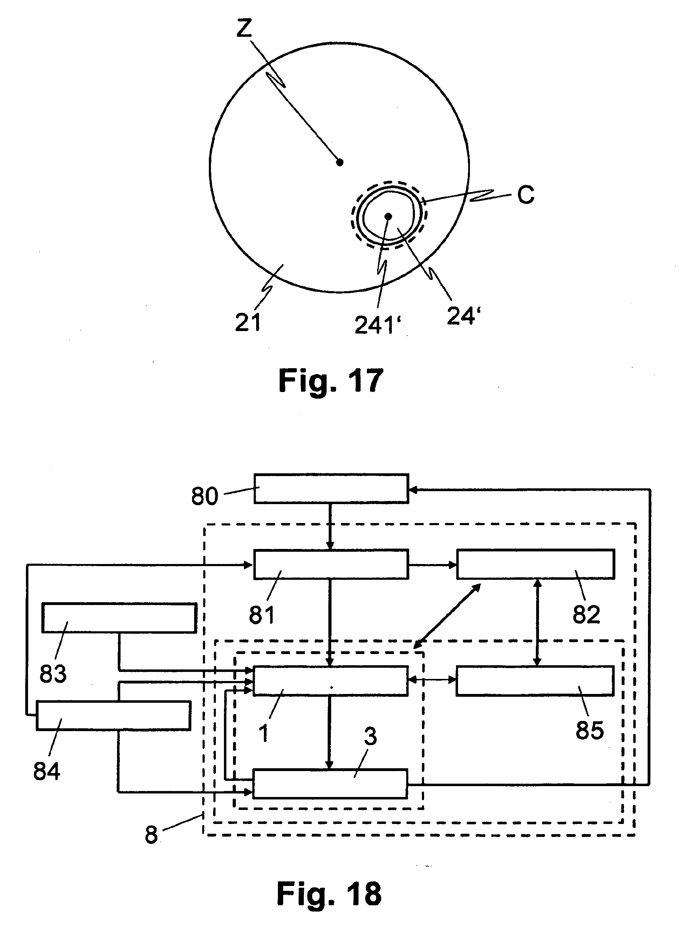

In der Figur 18 ist eine Anordnung des Computersystems 1 zum Generieren eines dreidimensionalen Schnittmusters als Teil eines (übergeordneten) computergestützten Operationsplanungssystems 8 dargestellt. Wie in der Figur 18 dargestellt ist umfasst das Operationsplanungssystems 8 zusätzlich zum Computersystem 1 mehrere weitere computergestützte Systeme und/oder Module, insbesondere ein Diagnose System 81, eine Datenbank 82, und ein Simulationsmodul 85. Das Diagnose System 81 wird von einem Arzt 84 gesteuert und erzeugt für das Computersystem 1 Diagnosedaten basierend auf Messungen und Daten an respektive von einem Patienten 80. Die Diagnosedaten werden in der Datenbank 82 gespeichert und dem Computersystem 1 zur Erzeugung eines dreidimensionalen Schnittmusters zugeführt. Die Erzeugung des Schnittmusters erfolgt gemäss Instruktionen des Arztes 84 basierend auf einem (dreidimensionalen) Augenmodell 83. Eine geplante Behandlung wird durch das Simulationsmodul 85 basierend auf dem definierten Schnittmuster und Laserdaten aus der Datenbank 82 simuliert, wobei neben der mechanischen Veränderung des Auges 2 insbesondere auch optische Eigenschaften des Auges 2 auf Grund der durch das Schnittmuster definierten refraktiven Korrektur des Auges 2 bestimmt werden. Zur eigentlichen Behandlung eines Auges 2 (von einem Spender oder am Patienten 80) wird das Schnittmuster vom Computersystem 1 der Laservorrichtung 3 zugeführt.

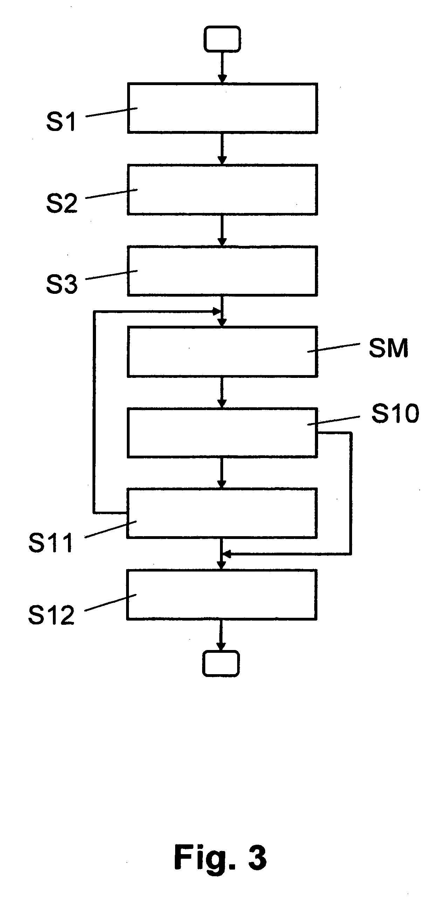

In den folgenden Abschnitten werden die verschiedenen Funktionen der funktionalen Module des Computersystems 1 mit Bezug zur Figur 3 beschrieben.

Nach dem Aufstarten des Computersystems 1 präsentiert das Steuermodul 10 dem Benutzer auf der Anzeige 17 eine grafische Benutzerschnittstelle 171, welche über die Eingabeelemente 16 bedienbar ist.

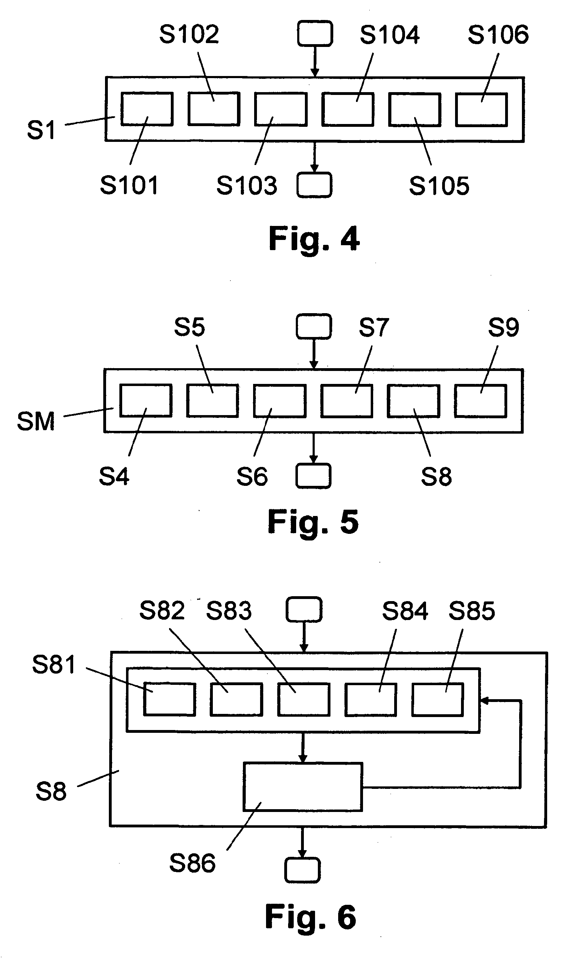

Im optionalen Schritt S1, führt das Steuermodul 10 benutzerselektierte Konfigurations- und Vorbereitungsschritte aus, welche nachfolgend mit Bezug zur Figur 4 beschrieben werden. Im Schritt S101 lädt das Steuermodul 10 Augendaten 181 für eines oder mehrere dreidimensionale Augenmodelle und speichert sie im Datenspeicher 18. Die Augenmodelle sind generische oder konkrete, patientenspezifische Augenmodelle. Die Augendaten 181 werden als bereitstehende Augendateien eingelesen oder über die Benutzerschnittstelle 171 z.B. in Form von Messwerten als Augenparameter definiert.

Im Schritt S102 lädt das Steuermodul 10 Laserdaten 182 für eine oder mehrere ophthalmologische Laservorrichtungen 3 und speichert sie im Datenspeicher 18. Die Laserdaten 182 werden als bereitstehende Laserdateien für bekannte Laservorrichtungen 3 eingelesen, über einen Kommunikationskanal 4 von der ophthalmologischen Laservorrichtung 3 gelesen und/oder über die Benutzerschnittstelle 171 in Form von eingegebenen Laserparametern definiert. Die Laserparameter umfassen Pulsenergie, Pulsbreite, maximale Pulsintensität (je nach Pulsform ergeben sich unterschiedliche Werte der Pulsintensität, bei sonst gleicher Pulsbreite und Pulsenergie), Pulsrate, Pulsabstände (Rasterdaten), Wellenlänge, Fokusgrösse, Fokusdistanz, mittlere Laserleistung und/oder numerische Apertur der ophthalmologischen Laservorrichtungen 3.

Im Schritt S103 lädt das Steuermodul 10 Applikationsdaten 183 für eines oder mehrere Applikationselemente 34 und speichert sie im Datenspeicher 18. Die Applikationsdaten 183 werden als bereitstehende Applikationsdateien für bekannte Applikationselemente 34 eingelesen, über einen Kommunikationskanal 4 von der ophthalmologischen Laservorrichtung 3 gelesen und/oder über die Benutzerschnittstelle 171 in Form von eingegebenen Applikationsparametern definiert. Die Applikationsparameter umfassen beispielsweise Angaben über Dicke, Form und/oder Transmissionscharakteristik.

Im Schritt S104 lädt das Steuermodul 10 Maskendaten 184 für eine oder mehrere Projektionsmasken 33 und speichert sie im Datenspeicher 18. Die Maskendaten 184 werden als bereitstehende Maskendateien für bekannte Projektionsmasken 33 eingelesen, über einen Kommunikationskanal 4 von der ophthalmologischen Laservorrichtung 3 gelesen und/oder über die Benutzerschnittstelle 171 in Form von eingegebenen Maskenparametern definiert. Die Maskenparameter umfassen beispielsweise Angaben über Form und Transmissionscharakteristik.

Im Schritt S105 lädt das Steuermodul 10 Flächenelementdaten 185 für mehrere Flächenelemente und speichert sie im Datenspeicher 18. Die Flächenelementdaten 185 werden vorzugsweise als bereitstehende Flächenelementdatei für eine Bibliothek respektive Liste von bekannten Flächenelementen eingelesen. Die Flächenelemente umfassen insbesondere Flächenelemente zweiter Ordnung, z.B. ebene Flächenelemente, konusförmige Flächenelemente, zylinderförmige Flächenelemente oder sphärische Flächenelement, sowie Splineflächenelemente und Flächenelemente, die durch eine Schnittlinie und eine Schnittlinientrajektorie definiert sind (in einer Variante umfasst die Schnittlinientrajektorie eine Verdrillung).

Im Schritt S106 lädt das Steuermodul 10 Schnittmusterdaten 186 für eines oder mehrere dreidimensionale Schnittmuster und speichert sie im Datenspeicher 18. Die Schnittmusterdaten 186 werden vorzugsweise als bereitstehende Schnittmusterdateien z.B. von einem Datenträger eingelesen oder mittels des Kommunikationsmoduls 19 über ein Telekommunikationsnetz von einem internetbasierten Webserver bezogen, welcher gespeicherte Schnittmusterdateien zur Wiederverwendung verfügbar mach.

Wie in der Figur 3 dargestellt ist, liest das Steuermodul 10 im Schritt S2 im Datenspeicher 18 die Augendaten 181 für das zu verwendende dreidimensionale Augenmodell. Falls Augendaten 181 für mehrere Augenmodelle gespeichert sind, wird vom Benutzer eine diesbezügliche Selektion angefragt und entgegengenommen. Vorzugsweise aktiviert das Steuermodul 10 das Visualisierungsmodul 13. Das Visualisierungsmodul 13 generiert basierend auf den selektierten Augendaten 181 das entsprechende dreidimensionale Augenmodell und stellt dieses in der Benutzerschnittstelle 171 auf der Anzeige 17 dar. Das dreidimensionale Augenmodell umfasst eine dreidimensionale Ansicht und/oder eine Aufsicht und eine Querschnittsansicht des Auges 2. In einer Variante ist das Visualisierungsmodul 13 eingerichtet auf der Anzeige 17 eine Visualisierung darzustellen, die der Sicht entspricht, die sich dem Benutzer während einer tatsächlichen Ausführung eines selektierten Schnittmusters durch Sichtfenster und/oder Monitore der Laservorrichtung 3 ergeben. Bei der Visualisierung werden dem dreidimensionalen Augenmodell vorzugsweise auch Merkmale überlagert, die durch die oben angeführten Messdaten und/oder Bilddaten definiert sind.

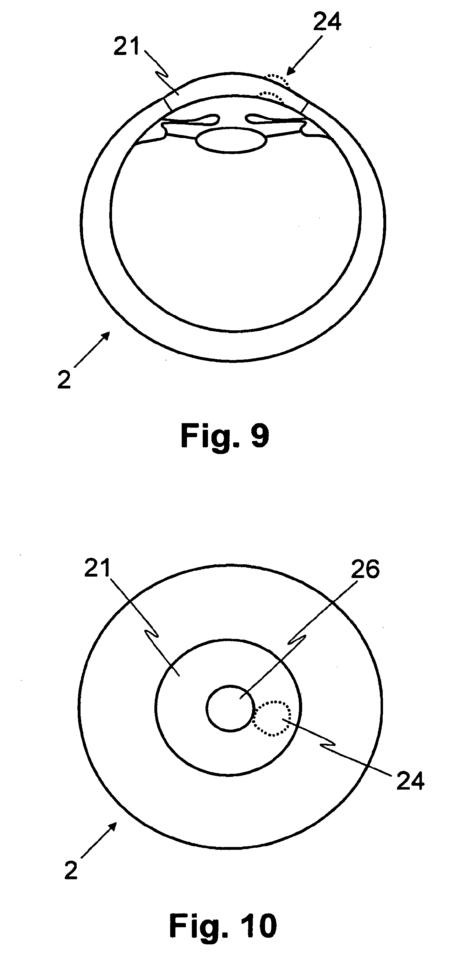

Figur 9 zeigt eine Querschnittsansicht des Auges 2, in welcher mit gestrichelter Linie schematisch ein Keratokonus 24 als Beispiel einer krankhaften Verdünnung und Ausstülpung der Cornea 21 (Hornhautverdünnung) illustriert ist.

Figur 10 zeigt eine Aufsicht des Auges 2, in welcher der Keratokonus 24 schematisch mit gestrichelter Linie auf der Cornea 21 illustriert ist, wobei der Keratokonus 24 im vorliegenden Beispiel die Iris 25 teilweise überlappt und an die Pupille 26 angrenzt.

Im optionalen Schritt S3, beispielsweise wenn vom Benutzer eine entsprechende Option selektiert und voreingestellt wurde, aktiviert das Steuermodul 10 den Referenzgenerator 113 zur automatischen Erzeugung einer Vorgabereferenz im dreidimensionalen Augenmodell. Beispielsweise abhängig von einer Voreinstellung, bestimmt der Referenzgenerator 113 basierend auf der optischen Achse der Laservorrichtung 3 oder auf charakteristischen Augenmerkmalen eine (oder mehrere) geometrische Referenz(en) im dreidimensionalen Augenmodell, beispielsweise ein Referenzpunkt, eine Referenzlinie, ein Referenzkreuz und/oder ein Referenzraster. Der Referenzgenerator 13 erzeugt in einer Variante zudem Konstruktionsraster, die z.B. auf Oberflächen von Augenstrukturen abgebildet werden und/oder diskretisiert Volumina. Als Beispiele seien hier Höhen- und Breitenlinien, Polar- und Kugelkoordinatenraster, aber auch hexagonale Gitter angeführt. Die geometrische(n) Referenz(en) wird (werden) im Datenspeicher gespeichert und durch das Visualisierungsmodul 13 auf der Anzeige 17 im dreidimensionalen Augenmodell dargestellt.

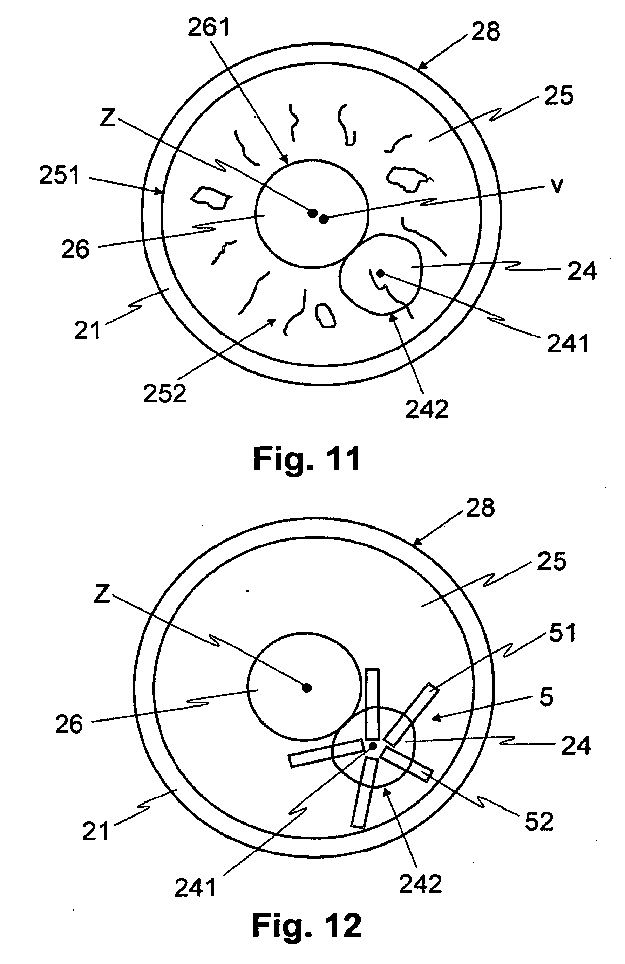

Figur 11 zeigt eine Aufsicht des Auges, in welcher die Iris 25 mit umlaufendem Limbus 28 dargestellt ist. In der Figur 11 sind verschiedene Beispiel von geometrischen Referenzen dargestellt, die durch den Referenzgenerator 113 generiert werden: die Pupille 26, der Pupillenrand 261, das Zentrum Z der Pupille 26, die optische oder visuelle Achse v, das Keratokonuszentrum 241, der Keratokonusrand 242, der Limbus 28, der Irisrand 251 und/oder charakteristische Merkmale des Irismusters 252. Die optische oder visuelle Achse v wird beispielsweise durch ein Messverfahren bestimmt, in welchem der Benutzer seinen Blick auf eine oder mehrere optische Referenzmarken richtet. Weitere Beispiele von geometrischen Referenzen umfassen die Linse 22, die dünnste Stelle der Cornea 21, ein Einschluss oder eine Narbe in der Cornea 21, die Position von Implantaten oder Adern in der Aderhaut und Strukturen der Retina 23.

Nach der Bestimmung des Augenmodells und gegebenenfalls einer automatisch generierten Referenz gibt das Steuermodul 10 dem Benutzer die Möglichkeit im Schritt SM ein dreidimensionales Schnittmuster zu definieren. Dazu stehen dem Benutzer über die Benutzerschnittstelle 171 verschiedene Editierfunktionen zur Verfügung, die nachfolgend mit Bezug zu den Figuren 5, 6 und 7 beschrieben werden.

Falls Laserdaten 182 für mehrere ophthalmologische Laservorrichtungen 3 gespeichert sind und/oder wenn der Benutzer über die Benutzerschnittstelle 171 eine Editierfunktion zum Selektieren und/oder Ändern einer bestimmten Laservorrichtung 3 wählt, aktiviert das Steuermodul 10 im Schritt S4 den Laserselektor 101 (siehe Figur 5). Wenn mehrere Laservorrichtungen 3 definiert sind, nimmt der Laserselektor 101 vom Benutzer eine diesbezügliche Selektion entgegen. Die Laserparameter der selektierten Laservorrichtung 3 werden dem Benutzer in der Benutzerschnittstelle 171 dargestellt, Änderungen werden entgegengenommen und die aktualisierten Laserdaten 182 im Datenspeicher 18 abgespeichert. Die Laserdaten 182 können vom Benutzer über die Benutzerschnittstelle 171 vor oder während einer Behandlung definiert werden, z.B. gemäss Aufforderung durch die Benutzerschnittstelle 171. Dabei werden dem Benutzer abhängig von den Laserdaten 182 der selektierten Laservorrichtung 3 Bereiche für die verschiedenen Laserparameter vorgegeben, in denen der Benutzer die Werte von benutzerdefinierbaren Laserparametern bestimmen kann. Die Laserparameter der selektierten Laservorrichtung 3 und insbesondere die benutzerdefinierten Laserparameter werden den Schnittmusterdaten 186 zugeordnet oder als Teil der Schnittmusterdaten 186 gespeichert. Zudem bestimmt der Laserselektor 101 basierend auf den aktuellen Laserdaten 182 der selektierten Laservorrichtung 3 die Form und die Abmessungen des projizierten Strahlkonus 32, welche als Strahlkonusdaten im Datenspeicher 18 gespeichert werden. Vorzugsweise wird durch das Visualisierungsmodul 13 auf der Anzeige 17 eine dreidimensionale Repräsentation des Strahlkonus 32 in der Benutzerschnittstelle 171 in Bezug zum dreidimensionalen Augenmodell grafisch dargestellt. In einer Variante stellt das Visualisierungsmodul 13 auch Masken und Saugringe und weitere chirurgische Hilfsmittel in Bezug zum dreidimensionalen Augenmodell auf der Anzeige 17 grafisch dar.

Wenn der Benutzer über die Benutzerschnittstelle 171 eine Editierfunktion zum Selektieren, Ändern und/oder Positionieren eines Applikationselements 34 wählt, aktiviert das Steuermodul 10 im Schritt S5 den Applikatorselektor 112. Der Applikatorselektor 112 zeigt dem Benutzer in der Benutzerschnittstelle 171 die verfügbaren Applikationselemente 34 an und nimmt eine diesbezügliche Selektion entgegen. Die Wahl des Applikationselements 34 wird im Datenspeicher 18 gespeichert und durch das Visualisierungsmodul 13 auf der Anzeige 17 in einem nicht applizierten Zustand dargestellt. Der Applikatorselektor 112 ist zudem eingerichtet vom Benutzer über die Eingabeelemente 16 Instruktionen zur relativen Positionierung des selektierten Applikationselements 34 zum dreidimensionalen Augenmodell, d.h. zur virtuellen Applikation auf dem Auge 2, entgegenzunehmen. Wenn die Positionierung des Applikationselements 34 einer Kontaktierung und Deformation des Auges 2 entspricht, wird das Deformationsmodul 12 aktiviert. Das Deformationsmodul 12 bestimmt basierend auf den aktuellen Augendaten 181 des selektierten Augenmodells, dem selektierten Applikationselement 34 und dessen relativen Position zum Auge 2 respektive zum Augenmodell ein deformiertes dreidimensionales Augenmodell, welches das Auge 2 im Zustand des aufgesetzten Applikationselements 34 repräsentiert. Das Visualisierungsmodul 13 stellt das deformierte dreidimensionale Augenmodell mit dem applizierten Applikationselement 34 in der Anzeige 17 dar. Somit wird dem Benutzer über die Benutzerschnittstelle eine flexible Selektion und Positionierung von Applikationselementen 34 auf einem Auge 2 im virtuellen, grafisch visualisierten Raum ermöglicht.

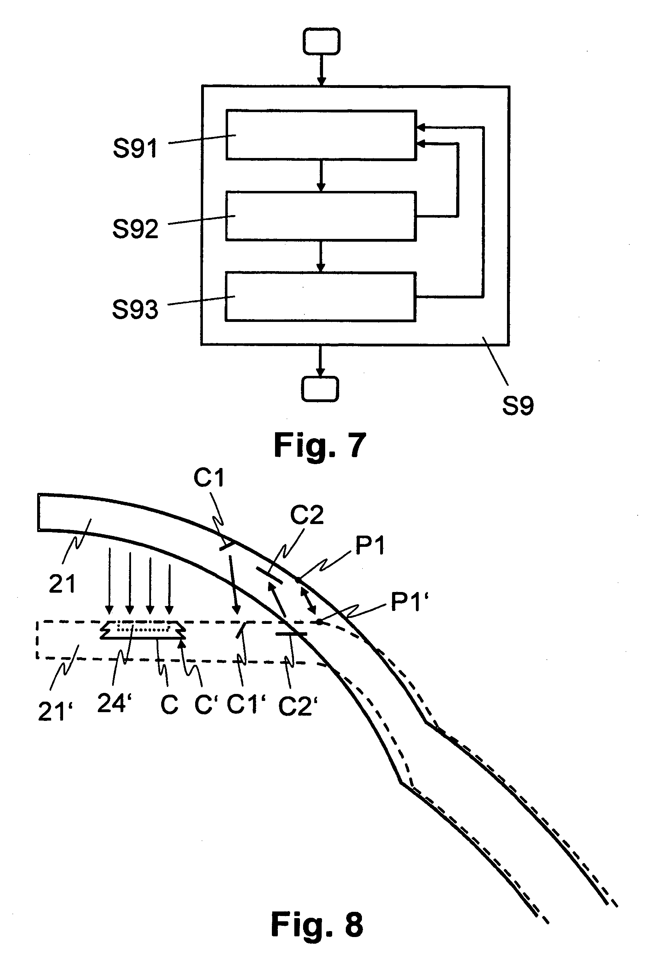

Figur 8 zeigt in der Querschnittsansicht schematisch einen Ausschnitt eines Beispiels einer Deformation der Cornea 21 durch Applanation. Dabei bezieht sich das Bezugszeichen 21' auf die gestrichelt illustrierte Cornea im deformierten Zustand.

Wenn der Benutzer über die Benutzerschnittstelle 171 eine Editierfunktion zum Selektieren, Ändern und/oder Positionieren einer Projektionsmaske 33 wählt, aktiviert das Steuermodul 10 im Schritt S6 den Maskenselektor 111. Der Maskenselektor 111 zeigt dem Benutzer in der Benutzerschnittstelle 171 die verfügbaren Projektionsmasken 33 an und nimmt eine diesbezügliche Selektion entgegen. Die Wahl der Projektionsmaske 33 wird im Datenspeicher 18 gespeichert und durch das Visualisierungsmodul 13 auf der Anzeige 17 dargestellt. Der Maskenselektor 111 ist zudem eingerichtet vom Benutzer über die Eingabeelemente 16 Instruktionen zur relativen Positionierung der selektierten Projektionsmaske 33 zum dreidimensionalen Augenmodell, d.h. zur virtuellen Positionierung vor das Auge 2, entgegenzunehmen. In einer Option wird die Projektionsmaske 33 automatisch auf ein selektiertes Applikationselement 34 aufgesetzt. In einer Ausführungsvariante ermöglicht der Maskenselektor 111 dem Benutzer zudem die Abmessungen und/oder Transmissionsparameter der lichtundurchlässigen oder lichtdämpfenden Maskenbereiche 33' zu ändern und als geänderte Projektionsmaske 33 im Datenspeicher 18 abzuspeichern. Das Visualisierungsmodul 13 stellt das dreidimensionale Augenmodell mit der positionierten und gegebenenfalls geänderten Projektionsmaske 33 in der Anzeige 17 dar. Somit wird dem Benutzer über die Benutzerschnittstelle eine flexible Selektion, Positionierung und Veränderung von Projektionsmasken 33 im virtuellen, grafisch visualisierten Raum ermöglicht. Zudem überprüft der Maskenselektor 111, ob sich durch die gewählte Projektionsmaske 33 eine Abschattung des durch die gespeicherten Strahlkonusdaten definierten Strahlkonus 32 ergibt, das heisst, ob eine fokussierte Einstrahlung der Femtosekundenlaserpulse durch die Projektionsmaske 33 verdeckt oder abgeschattet wird. Wenn eine maskenbedingte Abschattung detektiert wird, zeigt der Maskenselektor 111 dies dem Benutzer über die Benutzerschnittstelle 171 an und erwartet im Schritt S6 die Selektion einer alternativen Projektionsmaske 33 oder eine Korrektur der Maskenbereiche 33'. In einer Ausführungsvariante erzeugt der Maskenselektor 111 (z.B. auf eine entsprechende Benutzerinstruktion) automatisch eine korrigierte Projektionsmaske 33, welche keine Abschattungen bewirkt, und stellt diese dem Benutzer auf der Benutzerschnittstelle 171 dar.

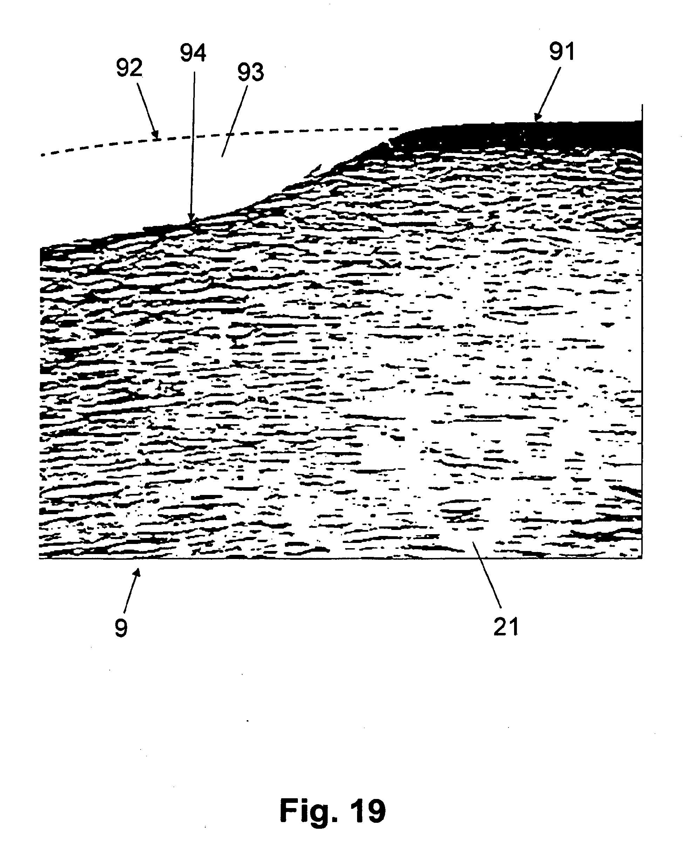

In Anlehnung an Figur 8 zeigt die Figur 19 ein Querschnittbild eines Beispiels eines Corneasegments 9 im undeformierten Zustand, nachdem im deformierten Zustand ein Corneaflap 93 (Cornealappen) mit einer ebenen Schnittfläche erzeugt wurde. Die durch das Epithel 91 begrenzte ursprüngliche Oberfläche der Cornea 21 ist in der Figur 19 durch die mit dem Bezugszeichen 92 bezeichnete gestrichelte Linie dargestellt. Das Beispiel zeigt den Einfluss der Deformation auf das Schnittergebnis. Die Schnittfläche 94, die im deformierten Zustand eben ist, ist im undeformierten Zustand gekrümmt.

Wenn der Benutzer über die Benutzerschnittstelle 171 eine Editierfunktion zum (manuellen) Definieren und/oder Ändern einer geometrischen Referenz wählt, aktiviert das Steuermodul 10 im Schritt S7 den Referenzgenerator 113. Der Referenzgenerator 113 zeigt dem Benutzer in der Benutzerschnittstelle 171 verschiedene verfügbare Typen von geometrischen Referenzen an und nimmt eine diesbezügliche Selektion entgegen. Ein ausgewählter Referenztyp wird durch das Visualisierungsmodul 13 auf der Anzeige 17 dargestellt. Wie bereits früher erwähnt, werden gewisse Typen von Referenzen vom Referenzgenerator 113 automatisch generiert und im ursprünglichen oder deformierten Augenmodell positioniert. Geometrische Referenzen können jedoch vom Benutzer über die Benutzerschnittstelle 171 auch manuell definiert und im unverformten oder deformierten Augenmodell positioniert werden, beispielsweise durch Eingabe von Positionskoordinaten oder durch Verschieben einer grafischen Repräsentation der Referenz mittels der Positionierungselemente. Überdies können geometrische Referenzen auch durch eine (visualisierte) Überlagerung des Augenmodells mit externen Messdaten und/oder Bilddaten definiert werden, die beispielsweise die Hauptkrümmungsachse bei Astigmatismus, die dünnste gemessene Stelle der Cornea 21, einen Einschluss oder eine Narbe in der Cornea 21 oder Implantate definieren. Eine definierte geometrische Referenz wird den betreffenden Augendaten 181 zugeordnet im Datenspeicher 18 gespeichert und durch das Visualisierungsmodul 13 im unverformten oder deformierten dreidimensionalen Augenmodell auf der Anzeige 17 dargestellt. Somit wird dem Benutzer über die Benutzerschnittstelle eine flexible Definition von geometrischen Referenzen im (unverformten und/oder deformierten) dreidimensionalen Augenmodell ermöglicht.

Figur 12 zeigt eine Aufsicht des Auges, in welcher die Iris 25 mit umlaufendem Limbus 28 dargestellt ist. Die Figur 12 illustriert schematisch ein Beispiel in welchem das Zentrum Z der Pupille 26 und das Keratokonuszentrum 241 als geometrische Referenzen ausgewählt wurden. Wie in der Figur 12 im Vergleich zur Figur 11 ersichtlich ist, reduziert das Visualisierungsmodul 13 die (dreidimensionale) Darstellung des Augenmodells auf die ausgewählten geometrischen Referenzen und filtert andere mögliche geometrischen Referenzen wie optische/visuelle Achse v oder Irismuster 27 aus, so dass die Darstellung für den Benutzer übersichtlicher wird.

Wenn der Benutzer über die Benutzerschnittstelle 171 eine Editierfunktion zum Definieren, Ändern und/oder Positionieren eines Schnitts wählt, aktiviert das Steuermodul 10 im Schritt S8 den Schnittflächeneditor 114. Der Schnittflächeneditor 114 bietet dem Benutzer die Möglichkeit Schnitte gemäss verschiedenen Schnittklassen zu definieren: Schnitte mit einer als Fläche zweiter Ordnung definierten Schnittfläche, Schnitte mit einer als Splinefläche definierten Schnittfläche, Schnitte mit einer durch Schnittlinie und Schnittlinientrajektorie definierten Schnittfläche und Schnitte mit einer durch ein gespeichertes dreidimensionales Schnittmuster definierten Schnittfläche.

Nachfolgend werden die Funktionen des Schnittflächeneditors 114 mit Bezug zur Figur 6 detaillierter beschrieben.

Wenn der Benutzer die Editierfunktion zum Definieren eines Schnitts mit einer durch ein gespeichertes dreidimensionales Schnittmuster definierten Schnittfläche wählt, zeigt der Schnittflächeneditor 114 im Schritt S81 dem Benutzer in der Benutzerschnittstelle 171 die durch die Schnittmusterdaten 185 definierten, verfügbaren Schnittmuster an und nimmt eine diesbezügliche Selektion entgegen. Die durch das selektierte Schnittmuster definierte Schnittfläche wird durch das Visualisierungsmodul 13 im dreidimensionalen Augenmodell auf der Anzeige 17 dargestellt, dabei erfolgt die Positionierung und Orientierung des selektierten Schnittmusters in Bezug zur definierten (bevorzugten respektive zugeordneten) geometrischen Referenz des Augenmodells. Wenn das selektierte Schnittmuster über die Definition von Schnittflächen hinaus editiert werden soll (z.B. Löschen oder Hinzufügen einer Schnittfläche, oder Ändern von geometrischer Referenz, Ausführungssequenz Applikationselement, Projektionsmaske und/oder Strahlkonus), werden die diesbezüglichen weiteren Funktionen des Schnittmustereditors 11 aktiviert. In einer Ausführungsvariante aktiviert der Schnittflächeneditor 114 im Schritt S81 den Schnittaufzeichner 115, welcher eingerichtet ist, basierend auf Benutzerinstruktionen im visualisierten dreidimensionalen Augenmodell durch Bewegen des durch die Strahlkonusdaten definierten Strahlkonus 32 einen virtuellen Gewebeschnitt zu erzeugen. Der virtuelle Gewebeschnitt wird als dreidimensionales Schnittmuster im Datenspeicher 18 gespeichert und durch das Visuallsierungsmodul 13 auf der Anzeige 17 dargestellt.

In der Figur 12 ist schematisch in der Aufsicht ein Schnittmuster 5 zur Korrektur eines Keratokonus 24 dargestellt. Das Schnittmuster 5 umfasst mehrere in die Cornea 21 geschnittene Taschen 51, 52, die jeweils durch eine separate Schnittfläche erzeugt werden. Die Taschen 51, 52 werden geschnitten um ein stabilisierendes Material einzufügen, z.B. durch Einspritzen von Riboflavin (Vitamin B2), das unter UV Licht polymerisiert. Die Schnittmusterdaten 186 des Schnittmusters 5 umfassen vorzugsweise eine Funktionsbezeichnung, hier. z.B. Stützung eines Keratokonus 24 mittels polymerisierender Flüssigkeit, welche einen für die Positionierung und Orientierung des Schnittmusters 5 bevorzugt verwendete geometrische Referenz des Augenmodells bestimmen, hier z.B. das Keratokonuszentrum 241 oder der Keratokonusrand 242. Der Schnittmustereditor 11 respektive der Schnittflächeneditor 114 positioniert das Schnittmuster 5 in Bezug zur bevorzugten respektive zugeordneten geometrischen Referenz (Default-Positionierung).

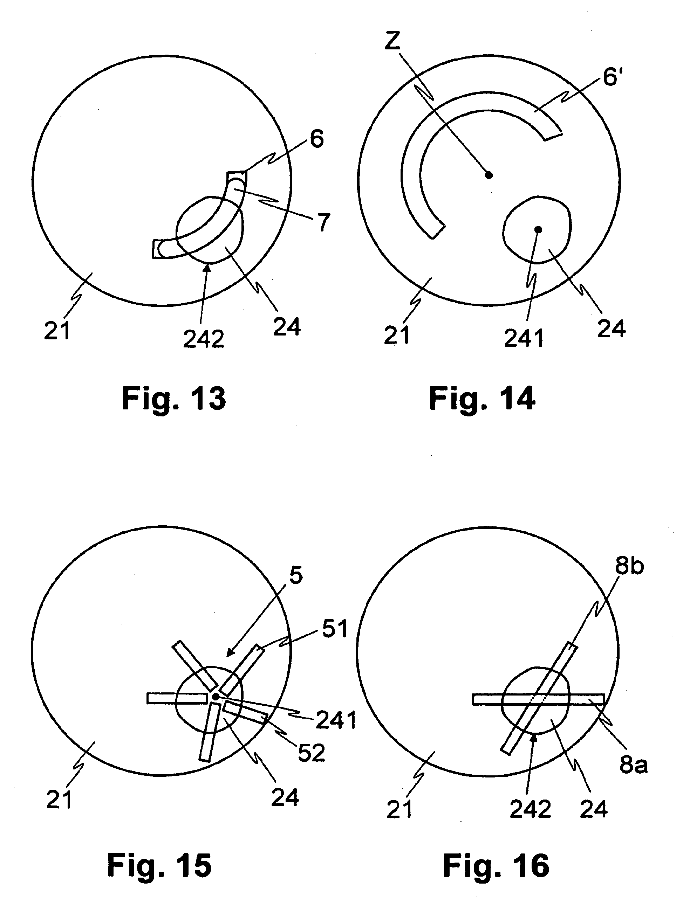

In den Figuren 13, 14, 15, 16 und 17 sind weitere Beispiele von Schnittmustern respektive definierten Schnittflächen in der Aufsicht der Cornea 21 illustriert.

Die Figur 13 illustriert ein Schnittmuster respektive eine durch eine definierte Schnittfläche bestimmte, in die Cornea 21 geschnittene Tasche 6 zur Stützung des Keratokonus 24 mittels eines Implantats 7 (mechanische Stützeinlage), welche in die geschnittene Tasche 6 eingeführt wird, z.B. ein so genanntes Intac aus Kunststoff. Im Beispiel der Figur 13 ist diesem Schnittmuster respektive dieser Schnittfläche der Keratokonusrand 242 als bevorzugte (Default) geometrische Referenz zugeordnet. Vorzugsweise wird das Schnittmuster respektive die durch die definierte Schnittfläche bestimmte Tasche 6 zum Keratokonusrand 242 zentriert angeordnet, wobei die Krümmung der Tasche 6 beispielsweise durch einen Kreisbogen um das Pupillenzentrum Z definiert ist.

Die Figur 14 illustriert ein Schnittmuster respektive eine durch eine definierte Schnittfläche bestimmte, in die Cornea 21 geschnittene Tasche 6' zum Glattziehen der durch das Keratokonus 24 erzeugten Ausbeulung der Cornea 21 mittels eines nicht gezeigten Implantats, das in die durch die Schnittfläche erzeugte Tasche 6' eingeführt wird. Im Beispiel der Figur 14 sind diesem Schnittmuster respektive dieser Schnittfläche das Pupillenzentrum Z und das Keratokonuszentrum 241 als bevorzugte (Default) geometrische Referenzen zugeordnet. Vorzugsweise wird das Schnittmuster respektive die durch die definierte Schnittfläche bestimmte Tasche 6' auf der dem Keratokonuszentrum 241 gegenüberliegenden Seite des Pupillenzentrums Z angeordnet, wobei die Krümmung der Tasche 6' beispielsweise durch einen Kreisbogen um das Pupillenzentrum Z definiert ist.

Die Figur 15 illustriert das obenstehend mit Bezug zur Figur 12 beschriebene, mehrere in die Cornea 21 geschnittene Taschen 51, 52 umfassende Schnittmuster 5 zur Korrektur des Keratokonus 24. Im Beispiel der Figur 15 ist dem Schnittmuster das Keratokonuszentrum 241 als bevorzugte (Default) geometrische Referenz zugeordnet. Vorzugsweise wird das Schnittmuster respektive die durch die definierten Schnittflächen bestimmten Taschen 51, 52 sternförmig zum Keratokonuszentrum 241 angeordnet, wobei die Einstiche respektive Öffnungen der Taschen 51, 52 jeweils auf der vom Keratokonuszentrum 241 abgewandten Seite der Taschen 51, 52 angebracht sind.

Die Figur 16 illustriert ein Schnittmuster mit zwei jeweils durch eine definierte Schnittfläche bestimmten, in die Cornea 21 geschnittenen, kreuzförmig, mit einem Abstand übereinanderliegenden Taschen 8a, 8b zur Stützung des Keratokonus 24 mittels mechanischen Stützeinlagen, welche in die geschnittenen Taschen 8a, 8b eingeführt werden. Im Beispiel der Figur 16 ist diesem Schnittmuster respektive diesen Schnittflächen der Keratokonusrand 242 als bevorzugte (Default) geometrische Referenz zugeordnet. Vorzugsweise wird das Schnittmuster respektive die durch die Schnittflächen definierte Kreuzform zum Keratokonusrand 242 zentriert angeordnet.

In der Figur 17 ist schematisch in der Aufsicht ein Schnittmuster C für eine lokale Keratoplastik dargestellt. Bei einer herkömmlichen Corneatransplantation wird ein wesentlicher Teil der Cornea 21 (beispielsweise >50% der Cornea) durch eine zentral aus einer Spendercornea herausgeschnittene Corneascheibe ersetzt. Im Unterschied dazu wird bei der in der Figur 17 illustrierten lokalen Keratoplastik ein verletztes oder schadhaftes Corneastück 24', z.B. eine lokale Corneaverätzung oder Einschlüsse/Verletzungen in der Cornea 21, massgeschneidert in einem lokalen Bereich um die beschädigte Stelle herum ersetzt. In der Figur 8 ist das Schnittmuster C für die lokale Keratoplastik im Querschnitt dargestellt. Wie in der Figur 8 ersichtlich ist, ist das Schnittmuster C so dimensioniert und angeordnet, dass es das schadhafte Corneastück 24' vollständig umfasst. Das Schnittmuster C umfasst mehrere in die Cornea 21 geschnittene Schnittflächen, welche einen (ausgeschnittenen) Zylinder definieren, bei dem der umlaufende Randbereich C' ein Profil für eine formschlüssige Aufnahme eines entsprechend geformten Corneaersatzstücks aufweist, was einen besseren Halt des Ersatzstücks in der Patientencornea ermöglicht. Das Profil des Randbereichs C' ist beispielsweise sägezahnförmig gezackt oder abgestuft. Die Schnittmusterdaten 186 des Schnittmusters C umfassen vorzugsweise eine Funktionsbezeichnung, hier. z.B. lokale Keratoplastik, welche einen für die Positionierung und Orientierung des Schnittmusters C bevorzugt verwendete geometrische Referenz des Augenmodells bestimmen, hier z.B. das Zentrum 241' des schadhaften Corneastücks 24'. Um eine sequenzbasierte Abschattung zu vermeiden werden für die lokale Keratoplastik die von der Corneaoberfläche abgewandten unteren Schnittflächen des Schnittmusters C vor den der Corneaoberfläche zugewandten oberen Schnittflächen des Schnittmusters C geschnitten. An dieser Stelle soll zudem auch festgehalten werden, das das Computersystem 1 und die damit verbundene Laservorrichtung 3 nicht nur für die Erzeugung und Bearbeitung von Schnittmustern an Patientenaugen einsetzbar ist, sondern insbesondere auch für das Herstellen von Ersatzcorneastücken aus Spendercorneas respektive von Implantaten geeignet und einsetzbar ist. Die lokale Keratoplastik hat insbesondere auch den Vorteil, dass eine Spendercornea für die Herstellung von mehreren Ersatzcorneastücken für einen oder mehrere Patienten verwendbar ist.

Wenn der Benutzer die Editierfunktion zum Definieren eines Schnitts mit einer durch Schnittlinie und Schnittlinientrajektorie definierten Schnittfläche wählt, zeigt der Schnittflächeneditor 114 im Schritt S82 dem Benutzer in der Benutzerschnittstelle 171 grafische Hilfsmittel zur Definition einer Schnittlinie im visualisierten dreidimensionalen Augenmodell an, beispielsweise einzelne oder mehrere gerade Liniensegmente zwischen jeweils zwei benutzerdefinierten Punkten, oder eine gekrümmte Linie durch mehrere benutzerdefinierte Punkte, wobei die Punkte jeweils durch eine dreidimensionale Position im dreidimensionalen Augenmodell definiert sind. Die einzelnen Punkte werden durch den Benutzer im visualisierten dreidimensionalen Augenmodell aber vorzugsweise auf einer Oberfläche definiert, beispielsweise auf der Cornea 21, auf der Linse 22, auf der Retina 23 oder auf einem definierten Meridianschnitt durch das Augengewebe. Die definierte Schnittlinie wird durch das Visualisierungsmodul 13 im dreidimensionalen Augenmodell auf der Anzeige 17 dargestellt. Der Schnittflächeneditor 114 ist zudem eingerichtet vom Benutzer über die Eingabeelemente 16 Instruktionen zur Definition einer Schnittlinientrajektorie im dreidimensionalen Augenmodell sowie zur Änderung der Schnittlinie entgegenzunehmen. Die Schnittlinientrajektorie gibt an, in welcher Richtung die definierte Schnittlinie zur Ausführung des Schnitts im dreidimensionalen Augenmodel bewegt (z.B. verschoben oder gedreht) werden soll, und definiert somit eine auf der Schnittlinie basierende Schnittfläche.

Wenn der Benutzer die Editierfunktion zum Definieren eines Schnitts mit einer als Fläche zweiter Ordnung oder als Splinefläche definierten Schnittfläche wählt, zeigt der Schnittflächeneditor 114 im Schritt S83 dem Benutzer in der Benutzerschnittstelle 171 die durch die Flächenelementdaten 185 definierten, verfügbaren Flächenelemente zweiter Ordnung an und nimmt eine diesbezügliche Selektion entgegen. Das gewählte Flächenelement wird durch das Visualisierungsmodul 13 auf der Anzeige 17 dargestellt. Der Schnittflächeneditor 114 ermöglicht dem Benutzer zudem Schnittflächen automatisch äquidistant zu einer bestimmten Oberfläche zu definieren, beispielsweise in Bezug zu selektierten Augenstrukturen und/oder Applikationselementen.

Wenn der Benutzer über die Benutzerschnittstelle 171 eine Editierfunktion zum Ändern einer Schnittfläche wählt, nimmt der Schnittflächeneditor 114 im Schritt S84 vom Benutzer über die Eingabeelemente 16 Instruktionen zur Änderung einer selektierten Schnittfläche im dreidimensionalen Augenmodell entgegen. Der Schnittflächeneditor 114 ermöglicht dem Benutzer insbesondere zur Änderung einer Schnittfläche ein ausgewähltes Flächenelement respektive eine Schnittlinie oder ein Schnittmuster in der Grösse zu skalieren, die Schnittfläche entlang einer oder mehreren Grenzlinien zu begrenzen, die Krümmung der Schnittfläche zu verändern, die Orientierung der Schnittfläche zu verändern und/oder die Schnittrichtung der Schnittfläche zu definieren respektive zu ändern. Die Schnittrichtung bestimmt für eine Schnittfläche die Richtung, in der das Pulsraster der Femtosekundenlaserpulse aufgebaut wird. Bei einem Zylinderschnitt kann das Pulsrater beispielsweise so definiert werden, dass der Schnitt, z.B. spiralförmig, von unten nach oben ausgeführt wird, oder dass, wie bei einer Stichsäge oder einem Messer, von unten nach oben ausgeführte Vertikalschnitte, jeweils nacheinander im Kreis gefahren werden. Dieses Beispiel zeigt die durch die Femtosekundenlasertechnologie ermöglichte Flexibilität, wobei Abschattungsverhältnisse und Schnittzeit bei diesen Schnittführungen deutlich unterschiedlich und vom Computersystem optimierbar sind, wie später beschrieben wird. In einer Variante ist es auch möglich zusätzlich zur Schnittrichtung einer Schnittfläche auch einen Startpunkt (Ausgangspunkt) für das Pulsraster der Femtosekundenlaserpulse zu definieren, z.B. durch Wahl von einem aus mehreren für die betreffende Schnittfläche definierten Startpunktmöglichkeiten.

Wenn der Benutzer über die Benutzerschnittstelle 171 eine Editierfunktion zum Positionieren einer Schnittfläche im (unverformten und/oder deformierten) dreidimensionalen Augenmodell wählt, nimmt der Schnittflächeneditor 114 im Schritt S85 vom Benutzer über die Eingabeelemente 16 Instruktionen zur Änderung der relativen Position bezüglich der geometrischen Referenz entgegen. Die relative Position wird beispielsweise durch Eingabe von Koordinaten oder vorzugsweise unter Verwendung der Positionierungselemente durch grafisches Verschieben der betreffenden Schnittfläche im (unverformten oder deformierten) dreidimensionalen Augenmodell definiert. Beispielsweise können zusätzlich zu der Eingabe von x/y-Koordinaten über eine Computermaus, eines Touchpads oder Trackballs z-Koordinaten mittels eines zusätzlichen Bedienelements, z.B. ein Scrollwheel, definiert werden. Die Positionierung erfolgt direkt in einer dreidimensionalen grafischen Darstellung und/oder in einer kombinierten Darstellung in der Aufsicht und im Querschnitt des dreidimensionalen Augenmodells (siehe dazu auch die nachfolgenden Ausführungen zur Figur 8).

Wenn der Benutzer dem Schnittflächeneditor 114 über die Benutzerschnittstelle 171 anzeigt, dass die Definition, Änderung und/oder Positionierung des Schnitts beendet ist, speichert der Schnittflächeneditor114 im Schritt S86 die entsprechenden Schnittdaten im Datenspeicher 18.

An dieser Stelle soll festgehalten werden, dass das Steuermodul 10 einem Benutzer ermöglicht, Schnitte mit dem Schnittflächeneditor 114 zu definieren und zu positionieren, bevor mit dem Applikatorselektor 112 ein Applikationselement 34 gewählt und positioniert wird. Das Deformationsmodul 12 ist eingerichtet, basierend auf dem durch die definierten Schnitte bestimmten dreidimensionalen Schnittmuster und dem gewählten und positionierten Applikationselement 34, ein deformiertes dreidimensionales Schnittmuster zu bestimmen, in welchem die im dreidimensionalen Augenmodell positionierten Schnittflächen zusammen mit der Deformation des Augenmodells entsprechend in einen deformierten Zustand mitdeformiert werden, so dass Punkte der deformierten Schnittflächen jeweils durch einen transformierten Punkt des deformierten Augenmodells definiert sind, der auf dem ursprünglichen gemeinsamen Punkt des unverformten Augenmodells und der unverformten Schnittfläche beruht. Der Benutzer kann somit Schnittflächen im unverformten Zustand des virtuellen Auges definieren und durch virtuelle Applikation des Applikationselements 34 automatisch in einen deformierten Zustand des Auges transformieren lassen.

Figur 8 zeigt im Querschnitt einen Teilbereich der undeformierten Cornea 21 und der deformierten Cornea 21' und darin positionierte Schnittlinien C1, C2, C1', C2' (respektive Schnittflächen) und Schnittpunkte P1, P1'. Wie in der Figur 8 schematisch illustriert ist, wird die Schnittlinie C1 im undeformierten Zustand der Cornea 21 positioniert und bei einer Deformation der Cornea 21 (durch das Deformationsmodul 12) in die Schnittlinie C1' im deformierten Zustand der Cornea 21' transformiert. Umgekehrt wird die Schnittlinie C2' im deformierten Zustand der Cornea 21' positioniert und beim Rückgängigmachen der Deformation (durch das Deformationsmodul 12) in die Schnittlinie C2 im undeformierten Zustand der Cornea 21 transformiert. In entsprechender Weise wird auch ein einzelner Schnittpunkt P1, P1' durch eine Deformation respektive durch Rückgängigmachen der Deformation (durch das Deformationsmodul 12) von einer Position im undeformierten Zustand der Cornea 21 in eine Position im deformierten Zustand der Cornea 21' respektive umgekehrt transformiert. Durch die Deformation respektive umgekehrte Transformation werden gerade Linien im Allgemeinen gedreht, gestreckt/gestaucht und gebogen (siehe auch Figur 19).

Wenn für ein dreidimensionales Schnittmuster mehrere Schnitte definiert wurden, schaltet das Steuermodul 10 dem Benutzer die Editierfunktion zum Definieren einer Ausführungssequenz frei. Wenn der Benutzer über die Benutzerschnittstelle 171 die Editierfunktion zum Definieren der Ausführungssequenz wählt, aktiviert das Steuermodul 10 im Schritt S9 den Sequenzgenerator 116. In einer Ausführungsvariante wird der Sequenzgenerator 116 im Schritt S10 automatisch durch den Schnittmustergenerator 117 aktiviert.

Nachfolgend werden die Funktionen des Sequenzgenerators 116 mit Bezug zur Figur 7 detaillierter beschrieben.

Im Schritt S91 zeigt der Sequenzgenerator 116 dem Benutzer über die Benutzerschnittstelle 171 die aktuelle Ausführungssequenz der definierten Schnitte dar. Dabei wird ohne Benutzerinstruktionen eine anfängliche Ausführungssequenz definiert, welche der (zeitlichen) Reihenfolge entspricht, in der diese Schnitte definiert wurden. Der Sequenzgenerator 116 nimmt vom Benutzer über die Benutzerschnittstelle 171 Instruktionen zum Ändern und/oder Übernehmen der aktuellen oder geänderten Ausführungssequenz entgegen. Der Benutzer kann auch Stopppunkte für die Simulation und/oder die tatsächliche Behandlung setzen und mit den Schnittmusterdaten 186 speichern. Die Stopppunkte erlauben dem Benutzer beispielsweise während einer Simulation und/oder tatsächlichen Behandlung Änderungen an den Schnittmusterdaten und insbesondere an den (benutzerdefinierbaren) Laserparametern vorzunehmen.

In den Schritten S92 und S93 führt der Sequenzgenerator 116 für die im Schritt S91 definierte Ausführungssequenz eine Plausibilitätsprüfung durch. Die Plausibilitätsprüfung umfasst in einer Variante auch die Detektierung von penetrierenden Schnitten, welche Augenstrukturen, beispielsweise die Oberfläche der Cornea 21 oder der Linse 22, durchdringen. Für detektierte penetrierende Schnitte wird dem Benutzer jeweils auf der Benutzerschnittstelle 171 eine Warnung angezeigt.

Im Schritt S92 überprüft der Sequenzgenerator 116, ob sich bei der Ausführung der Schnitte gemäss der definierten Ausführungssequenz eine Abschattung von später auszuführenden Schnitten durch vorher auszuführende Schnitte ergibt, das heisst, ob eine fokussierte Einstrahlung der Femtosekundenlaserpulse zur Erzeugung der später zu erzeugenden Schnittfläche durch den vorher zu erzeugenden Schnitt verdeckt oder abgeschattet wird. Für die Bestimmung der Verdeckung oder Abschattung wird untersucht, ob der durch die gespeicherten Strahlkonusdaten definierte Strahlkonus 32 bei der Ausführung eines späteren Schnitts durch einen früher erzeugten Schnitt unterbrochen wird. Wenn eine sequenzbedingte Abschattung detektiert wird, zeigt der Sequenzgenerator 116 dies dem Benutzer über die Benutzerschnittstelle 171 an und erwartet im Schritt S91 eine Korrektur der Ausführungssequenz. In einer Ausführungsvariante erzeugt der Sequenzgenerator 116 automatisch eine korrigierte Ausführungssequenz (z.B. auf eine entsprechende Benutzerinstruktion), welche keine Abschattungen bewirkt, und stellt diese dem Benutzer auf der Benutzerschnittstelle 171 dar.

In einer Ausführungsvariante trennt der Sequenzgenerator 116 zudem sich überschneidende Schnittflächen automatisch in separate Schnittflächen, die dann in der Ausführungssequenz als separate Schnittflächen behandelt werden.

In einer weiteren Ausführungsvariante berücksichtigt der Sequenzgenerator 116 bei der Bestimmung der sequenzbedingten Abschattung respektive bei der Erzeugung einer korrigierten Ausführungssequenz die Resorption im Gewebe von Gas, das beim Gewebeabbau durch die Laserbehandlung entsteht. Dabei werden gespeicherte Resorptionszeitwerte verwendet, die die Zeitdauer für die Wiedererreichung der Transparenz im Gewebe nach dessen Behandlung angeben. Nach der Resorption verursacht ein durchgeführter Schnitt keine beeinträchtigende Abschattung und es können wieder Schnitte in darunterliegendem Gewebe durchgeführt werden.

In einer zusätzlichen Ausführungsvariante berücksichtigt und/oder ändert der Sequenzgenerator 116 bei der Bestimmung der sequenzbedingten Abschattung respektive bei der Erzeugung einer korrigierten Ausführungssequenz überdies die Schnittrichtung der Schnittflächen, d.h. die Richtung in der der Pulsraster der Femtosekundenlaserpulse aufgebaut wird. Dabei wird beispielsweise eine Optimierung vorgenommen, in der die vom Benutzer vorgegebene Ausführungssequenz, Schnittrichtung und/oder Startpunkt der Schnittflächen des Schnittmusters so angepasst werden, dass die Schnitte möglichst schnell und abschattungsfrei ausgeführt werden. In der Regel wird sowohl die Sequenz der Schnittflächen als auch das Pulsraster innerhalb einer Schnittfläche von unten nach oben ausgeführt, wobei der Scanrichtung mit der höchsten Vorschubgeschwindigkeit der Vorrang gegeben wird.

Wenn eine abschattungsfreie Ausführungssequenz vorliegt, überprüft der Sequenzgenerator 116 im optionalen Schritt S93, ob bei der Ausführung der Schnitte gemäss der definierten Ausführungssequenz eine erwartete thermische Belastung des Auges 2 einen definierten Grenzwert übersteigt. Dabei wird die erwartete thermische Belastung des Auges 2 beispielsweise wie in EP1810647 beschrieben bestimmt, wo ein Modellierungsmodul die (thermische) Belastungen der Augenstrukturen, die sich aus der Behandlung durch den Laserstrahl ergeben, basierend auf dem Augenmodell und der Lichtausbreitung im Auge sowie den definierten Laserparametern unter Berücksichtigung von Absorptionskoeffizienten, die den Augenstrukturen zugeordnet sind, ermittelt. Nach EP1810647 werden Gewebeschäden ausserhalb eines Schnittes im wesentlichen dadurch vermieden, dass die mittlere Bestrahlungsstärke im Schnittbereich (Bearbeitungsgebiet) reduziert und die maximalen Temperaturen im Gewebe ausserhalb des Schnitts reduziert werden, um Belastungsgrenzen von Augenstrukturen nicht zu überschreiten. Wenn eine sequenzbedingte thermische Überbelastung detektiert wird, zeigt der Sequenzgenerator 116 dies dem Benutzer über die Benutzerschnittstelle 171 an und erwartet im Schritt S91 eine Korrektur der Ausführungssequenz. In einer Ausführungsvariante erzeugt der Sequenzgenerator 116 automatisch eine korrigierte Ausführungssequenz (z.B. auf eine entsprechende Benutzerinstruktion), welche keine thermische Überbelastung bewirkt, und stellt diese dem Benutzer auf der Benutzerschnittstelle 171 dar. Wenn eine Ausführungssequenz ohne erwartete Überbelastung vorliegt, speichert der Sequenzgenerator 116 die definierte Ausführungssequenz ab.

Wenn der Benutzer dem Steuermodul 10 über die Benutzerschnittstelle 171 anzeigt, dass die Definition des dreidimensionalen Schnittmusters im Schritt SM abgeschlossen ist, erzeugt der Schnittmustergenerator 117 im Schritt S10 darauf basierend ein dreidimensionales Schnittmuster. Im Wesentlichen definiert das dreidimensionale Schnittmuster einen oder mehrere Gewebeschnitte durch eine oder mehrere in Bezug zu mindestens einer geometrischen Referenz im unverformten oder deformierten Augenmodell positionierte Schnittflächen, denen eine Ausführungssequenz zugeordnet ist oder die entsprechend der Ausführungssequenz geordnet sind. Die Schnittflächen sind im dreidimensionalen Schnittmuster beispielsweise in Form von Makroinstruktionen definiert. In einer Alternative oder zusätzlich umfasst das dreidimensionale Schnittmuster für jede Schnittfläche Pulsrasterinstruktionen zur Steuerung der Laservorrichtung 3. Mit den Schnittmusterdaten eines definierten Schnittmusters werden auch die (manuell oder automatisch) definierten geometrischen Referenzen an die Laservorrichtung 3 übertragen.

Wenn der Benutzer über die Benutzerschnittstelle 171 eine Simulationsfunktion zur Simulation des erzeugten Schnittmusters selektiert, aktiviert das Steuermodul 10 im Schritt S11 den Schnittsimulator 14, andernfalls werden die Schnittmusterdaten 186 des dreidimensionalen Schnittmusters im Schritt S12 im Datenspeicher 18 gespeichert.

Die Schnittmusterdaten 186 können zudem mittels des Kommunikationsmoduls 19 als Schnittmusterdateien über ein Telekommunikationsnetz an einen internetbasierten Webserver übermittelt und dort zur Wiederverwendung gespeichert werden.

Der Schnittsimulator 14 ist eingerichtet, die Ausführung der durch das dreidimensionale Schnittmuster definierten Gewebeschnitte zu simulieren und auf der Anzeige 17 zu visualisieren. Vorzugsweise wird das dreidimensionale Schnittmuster dabei in einem Schritt für Schritt Modus durchgeführt, wobei nach jedem Schritt vom Benutzer eine Instruktion zur Fortsetzung der Simulation mit dem nächsten Schritt, oder Instruktionen zur Selektion und Ausführung der verschiedenen Editierfunktionen (Schritt SM) zur Änderung des dreidimensionalen Schnittmusters respektive von darin enthaltenen Schnitten entgegengenommen werden. Dabei wird in einem Schritt, abhängig vom gewählten Simulationsmodus, die Ausführung einer Schnittfläche, einer Teilschnittfläche oder einer definierten Anzahl von Femtosekundenlaserpulsen simuliert und visualisiert. In einer Ausführungsvariante können vom Benutzer zudem auch Stopppunkte für die Simulation und/oder die tatsächliche Behandlung gesetzt werden, z.B. vor oder nach der Ausführung einer bestimmten Schnittfläche oder Teilschnittfläche, oder nach der Ausführung einer definierten Anzahl von Femtosekundenlaserpulsen. Das dreidimensionale Schnittmuster kann auch im Simulationsmodus (und in einer Variante auch während der tatsächlichen Behandlung) beispielsweise an einem Stopppunkt durch die Funktionen des Schnittmustereditors 11 geändert werden. Das dreidimensionale Schnittmuster kann insbesondere durch die folgenden Operationen geändert werden: Repositionieren einer Schnittfläche, Reorientieren einer Schnittfläche, Ändern einer Schnittrichtung einer Schnittfläche, Löschen einer Schnittfläche, Löschen eines Schnittflächenteils, Verändern einer Schnittfläche, Hinzufügen einer Schnittfläche, Duplizieren einer Schnittfläche, Ändern einer geometrischen Referenz, Ändern einer Ausführungssequenz für die Schnittflächen, Ändern eines beim Ausführen der Gewebeschnitte auf das Auge aufzusetzenden Applikationselements, Ändern einer beim Ausführen der Gewebeschnitte vor das Auge zu positionierenden Projektionsmaske und/oder Ändern eines durch fokussierte Einstrahlung der Femtosekundenlaserpulse definierten Strahlkonus 32. Überdies kann das Schnittmuster als Ganzes verändert werden, beispielsweise durch Skalieren (Grössenveränderung), (Re-) Orientieren und/oder Repositionieren (Verschieben).

Abschliessend soll angeführt werden, dass in der Beschreibung zwar Computerprogrammcode spezifischen funktionalen Modulen zugeordnet wurde und dass die Ausführung von Schritten in einer bestimmten Reihenfolge dargestellt wurde, dass der Fachmann jedoch verstehen wird, dass der Computerprogrammcode unterschiedlich strukturiert und die Reihenfolge von mindestens gewissen Schritten geändert werden kann, ohne dabei vom Schutzgegenstand abzuweichen.

## Patentansprüche

1. Computergestütztes System (1) zum Generieren eines dreidimensionalen Schnittmusters, welches einen oder mehrere mittels Femtosekundenlaserpulsen in einem menschlichen Auge (2) auszuführende Gewebeschnitte definiert, umfassend:
einen Datenspeicher (18) mit Augendaten (181), welche ein dreidimensionales Augenmodell definieren;
einen Referenzgenerator (113), welcher eingerichtet ist, mindestens eine geometrische Referenz mit Bezug zum dreidimensionalen Augenmodell zu definieren und zu speichern;
einen Schnittflächeneditor (114), welcher eingerichtet ist, basierend auf Benutzerinstruktionen mehrere Schnittflächen zu definieren und die mehreren Schnittflächen mit Bezug zur mindestens einen geometrischen Referenz im dreidimensionalen Augenmodell zu positionieren; und
einen Schnittmustergenerator (117), welcher eingerichtet ist, das dreidimensionale Schnittmuster zur Definition von Gewebeschnitten basierend auf den mehreren im dreidimensionalen Augenmodell durch den Benutzer bestimmten und positionierten Schnittflächen zu generieren und als Schnittmusterdaten (186) mit Makroinstruktionen oder Pulsrasterinstruktionen zur Steuerung einer ophthalmologischen Laservorrichtung (3) zu speichern.

2. System (1) nach Anspruch 1, wobei der Schnittflächeneditor (114) eingerichtet ist, die mindestens eine Schnittfläche basierend auf einer benutzerdefinierten Schnittlinie und einer benutzerdefinierten Schnittlinientrajektorie zu definieren.

3. System (1) nach einem der Ansprüche 1 oder 2, wobei der Schnittflächeneditor (114) eingerichtet ist, die mindestens eine Schnittfläche basierend auf mindestens einem Flächenelement zu definieren, welches basierend auf Benutzerinstruktionen aus einer Liste von mehreren unterschiedlichen Flächenelementen auswählbar ist.

4. System (1) nach einem der Ansprüche 1 bis 3, umfassend einen Applikatorselektor (112), welcher eingerichtet ist, basierend auf Benutzerinstruktionen ein beim Ausführen der Gewebeschnitte auf das Auge (2) aufzusetzendes Applikationselement (34) zu bestimmen; und ein Deformationsmodul (12), welches eingerichtet ist, basierend auf den Augendaten (181) und dem bestimmten Applikationselement (34), ein deformiertes dreidimensionales Augenmodell zu bestimmen, welches das Auge (2) im Zustand des aufgesetzten Applikationselements (34) repräsentiert.

5. System (1) nach Anspruch 4, wobei der Referenzgenerator (113) eingerichtet ist, die geometrische Referenz mit Bezug zum deformierten dreidimensionalen Augenmodell zu definieren und zu speichern; dass der Schnittflächeneditor (114) eingerichtet ist, die mindestens eine Schnittfläche mit Bezug zur mindestens einen geometrischen Referenz im deformierten dreidimensionalen Augenmodell zu positionieren; und dass der Schnittmustergenerator (117) eingerichtet ist, das dreidimensionale Schnittmuster basierend auf der mindestens einen oder mehreren im deformierten dreidimensionalen Augenmodell positionierten Schnittflächen zu generieren und zu speichern.

6. System (1) nach einem der Ansprüche 4 oder 5, wobei das Deformationsmodul (12) eingerichtet ist, basierend auf dem dreidimensionalen Schnittmuster und dem bestimmten Applikationselement (34), ein deformiertes dreidimensionales Schnittmuster zu bestimmen, welches auf einem deformierten Zustand der mindestens einen oder mehreren im dreidimensionalen Augenmodell positionierten Schnittflächen basiert, wobei der deformierte Zustand durch das deformierte dreidimensionale Augenmodell definiert ist.

7. System (1) nach einem der Ansprüche 1 bis 6, umfassend einen Sequenzgenerator (116), welcher eingerichtet ist, für die Schnittflächen der im dreidimensionalen Schnittmuster definierten Gewebeschnitte eine Ausführungssequenz automatisch oder basierend auf Benutzerinstruktionen zu bestimmen, wobei verhindert wird, dass eine gemäss der Ausführungssequenz früher zu erzeugende Schnittfläche eine fokussierte Einstrahlung der Femtosekundenlaserpulse zur Erzeugung einer gemäss der Ausführungssequenz später zu erzeugenden Schnittfläche verdeckt oder abschattet.

8. System (1) nach Anspruch 7, wobei der Sequenzgenerator (116) eingerichtet ist, eine Verdeckung oder Abschattung einer später zu erzeugenden Schnittfläche durch eine früher zu erzeugende Schnittfläche basierend auf einem durch fokussierte Einstrahlung der Femtosekundenlaserpulse definierten Strahlkonus (32) zu bestimmen.

9. System (1) nach einem der Ansprüche 7 oder 8, wobei der Sequenzgenerator (116) eingerichtet ist, die Ausführungssequenz für die Schnittflächen so zu bestimmen, dass eine erwartete thermische Belastung des Auges (2) einen definierten Grenzwert nicht übersteigt.

10. System (1) nach einem der Ansprüche 1 bis 9, umfassend einen Maskenselektor (111), welcher eingerichtet ist, basierend auf Benutzerinstruktionen eine beim Ausführen der Gewebeschnitte vor das Auge (2) zu positionierende Projektionsmaske (33) zu bestimmen, und basierend auf der bestimmten Projektionsmaske (33) und einem durch fokussierte Einstrahlung der Femtosekundenlaserpulse definierten Strahlkonus (32) zu überprüfen, ob die Schnittflächen der im dreidimensionalen Schnittmuster definierten Gewebeschnitte ohne Abschattung durch die Projektionsmaske (33) erzeugbar sind.

11. System (1) nach einem der Ansprüche 1 bis 10, umfassend ein Visualisierungsmodul (13), welches eingerichtet ist, basierend auf den Augendaten (181) eine Visualisierung des Auges (2) und einen durch fokussierte Einstrahlung der Femtosekundenlaserpulse definierten Strahlkonus (32) auf einer Anzeige (17) darzustellen; und einen Schnittaufzeichner (115), welcher eingerichtet ist, basierend auf Benutzerinstruktionen im visualisierten Auge (2) durch Bewegen des Strahlkonus (32) einen virtuellen Gewebeschnitt zu erzeugen und den virtuellen Gewebeschnitt als dreidimensionales Schnittmuster zu speichern.

12. System (1) nach einem der Ansprüche 1 bis 11, umfassend einen Schnittsimulator (14), welcher eingerichtet ist, basierend auf den Augendaten (181) und dem gespeicherten dreidimensionalen Schnittmuster eine Ausführung der durch das dreidimensionale Schnittmuster definierten Gewebeschnitte zu simulieren und auf einer Anzeige (17) zu visualisieren; und einen Schnittmustereditor (11), welcher eingerichtet ist, basierend auf Benutzerinstruktionen das gespeicherte dreidimensionale Schnittmuster durch mindestens eine Operation aus der folgenden Liste anzupassen: Repositionieren einer Schnittfläche, Reorientieren einer Schnittfläche, Ändern einer Schnittrichtung einer Schnittfläche, Löschen einer Schnittfläche, Löschen eines Schnittflächenteils, Verändern einer Schnittfläche, Hinzufügen einer Schnittfläche, Duplizieren einer Schnittfläche, Ändern einer geometrischen Referenz, Ändern einer Ausführungssequenz für die Schnittflächen, Ändern eines beim Ausführen der Gewebeschnitte auf das Auge (2) aufzusetzenden Applikationselements (34), Ändern einer beim Ausführen der Gewebeschnitte vor das Auge (2) zu positionierenden Projektionsmaske (33) und Ändern eines durch fokussierte Einstrahlung der Femtosekundenlaserpulse definierten Strahlkonus (32).

13. System (1) nach einem der Ansprüche 1 bis 12, wobei der Schnittmustergenerator (117) eingerichtet ist, im dreidimensionalen Schnittmuster Gewebeschnitte durch eine oder mehrere in Bezug zu einer geometrischen Referenz positionierte Schnittflächen zu definieren, wobei eine Schnittfläche jeweils durch mindestens einen Parameter aus der folgenden Liste definiert ist: Schnittlinie, Schnittlinientrajektorie, Schnittrichtung, Flächenelementklasse, Flächenelementabmessungen, Flächenelementkrümmung und Flächenelementorientierung, und wobei den Schnittflächen eine Ausführungssequenz zugeordnet ist.

14. System (1) nach einem der Ansprüche 1 bis 13, wobei der Schnittmustergenerator (117) eingerichtet ist, die Schnittflächen im dreidimensionalen Schnittmuster in Form von Makroinstruktionen zu definieren.

15. System (1) nach einem der Ansprüche 1 bis 14, wobei der Schnittflächeneditor (114) eingerichtet ist, basierend auf Benutzerinstruktionen die mindestens eine Schnittfläche durch mindestens eine Operation aus der folgenden Liste anzupassen: Skalieren der Grösse der Schnittfläche, Begrenzen der Schnittfläche entlang einer oder mehreren Grenzlinien, Verändern einer Krümmung der Schnittfläche, Ändern einer Orientierung der Schnittfläche, Ändern einer Schnittrichtung der Schnittfläche und Ändern einer Schnittklasse der Schnittfläche, wobei die Schnittklasse durch mindestens ein Element aus der folgenden Liste von Schnittklassen definiert ist: Schnitte mit einer als Fläche zweiter Ordnung definierten Schnittfläche, Schnitte mit einer als Splinefläche definierten Schnittfläche, Schnitte mit einer durch Schnittlinie und Schnittrichtung definierten Schnittfläche und Schnitte mit einer durch ein gespeichertes dreidimensionales Schnittmuster definierten Schnittfläche.

## Claims

1. Computer-based system (1) for generating a three-dimensional cut pattern defining one or more tissue cuts to be carried out in a human eye (2) by means of femtosecond laser pulses, comprising:
a data store (18) comprising eye data (181) defining a three-dimensional eye model;
a reference generator (113) configured to define and store at least one geometric reference in relation to the three-dimensional eye model;
a cut surface editor (114) configured to define a plurality of cut surfaces on the basis of user instructions and to position the plurality of cut surfaces in relation to the at least one geometric reference in the three-dimensional eye model; and
a cut pattern generator (117) configured to generate the three-dimensional cut pattern for defining tissue cuts on the basis of the plurality of cut surfaces positioned and determined by the user in the three-dimensional eye model and to store this as cut pattern data (186) with macro-instructions or pulse raster instructions for controlling an ophthalmological laser device (3).

2. System (1) according to Claim 1, wherein the cut surface editor (114) is configured to define the at least one cut surface on the basis of a user-defined cut line and a user-defined cut line trajectory.

3. System (1) according to either of Claims 1 or 2, wherein the cut surface editor (114) is configured to define the at least one cut surface on the basis of at least one two-dimensional element, which can be selected on the basis of user instructions from a list of a plurality of different two-dimensional elements.

4. System (1) according to one of Claims 1 to 3, comprising an applicator selector (112) configured to determine, on the basis of user instructions, an application element (34) to be placed onto the eye (2) when carrying out the tissue cuts; and a deformation module (12) configured to determine, on the basis of the eye data (181) and the specific application element (34), a deformed three-dimensional eye model which represents the eye (2) in the state of the applied application element (34).

5. System (1) according to Claim 4, wherein the reference generator (113) is configured to define and store the geometric reference in relation to the deformed three-dimensional eye model; wherein the cut surface editor (114) is configured to position the at least one cut surface in relation to the at least one geometric reference in the deformed three-dimensional eye model; and wherein the cut pattern generator (117) is configured to generate and store the three-dimensional cut pattern on the basis of the at least one or more cut surfaces positioned in the deformed three-dimensional eye model.

6. System (1) according to either of Claims 4 and 5, wherein the deformation module (12) is configured to determine, on the basis of the three-dimensional cut pattern and the specific application element (34), a deformed three-dimensional cut pattern, which is based on a deformed state of the at least one or more cut surfaces positioned in the three-dimensional eye model, wherein the deformed state is defined by the deformed three-dimensional eye model.

7. System (1) according to one of Claims 1 to 6, comprising a sequence generator (116) configured to determine, automatically or on the basis of user instructions, an application sequence for the cut surfaces of the tissue cuts defined in the three-dimensional cut pattern, with the process preventing a cut surface to be generated earlier in accordance with the application sequence from covering or shadowing focused incidence of the femtosecond laser pulses for generating a cut surface to be generated later in accordance with the application sequence.

8. System (1) according to Claim 7, wherein the sequence generator (116) is configured to determine covering or shadowing of a cut surface to be generated later by a cut surface to be generated earlier on the basis of a beam cone (33) defined by focused incidence of the femtosecond laser pulses.

9. System (1) according to either of Claims 7 or 8, wherein the sequence generator (116) is configured to determine the application sequence for the cut surfaces in such a way that an expected thermal load on the eye (2) does not exceed a defined limit value.

10. System (1) according to one of Claims 1 to 9, comprising a mask selector (111) configured to determine, on the basis of user instructions, a projection mask (33) to be positioned in front of the eye (2) when carrying out the tissue cuts, and, on the basis of the specific projection mask (33) and a beam cone (32) defined by focused incidence of the femtosecond laser pulses, to check whether the cut surfaces of the tissue cuts defined in the three-dimensional cut pattern can be generated without shadowing by the projection mask (33).

11. System (1) according to one of Claims 1 to 10, comprising a visualization module (13) configured, on the basis of the eye data (181), to display on a display (17) a visualization of the eye (2) and a beam cone (32) defined by focused incidence of the femtosecond laser pulses; and a cut recorder (115) configured, on the basis of user instructions, to generate a virtual tissue cut in the visualized eye (2) by moving the beam cone (32) and configured to store the virtual tissue cut as a three-dimensional cut pattern.

12. System (1) according to one of Claims 1 to 11, comprising a cut simulator (14) configured, on the basis of the eye data (181) and the stored three-dimensional cut pattern, to simulate carrying out the tissue cuts defined by the three-dimensional cut pattern and to visualize this on a display (17); and a cut pattern editor (11) configured to adapt, on the basis of user instructions, the stored three-dimensional cut pattern by at least one operation from the following list: repositioning a cut surface, reorienting a cut surface, changing a cut direction of a cut surface, deleting a cut surface, deleting a cut surface portion, modifying a cut surface, adding a cut surface, duplicating a cut surface, changing a geometric reference, changing an application sequence for the cut surfaces, changing an application element (34) to be placed onto the eye (2) when carrying out the tissue cuts, changing a projection mask (33) to be positioned in front of the eye (2) when carrying out the tissue cuts and changing a beam cone (32) defined by focused incidence of the femtosecond laser pulses.

13. System (1) according to one of Claims 1 to 12, wherein the cut pattern generator (117) is configured to define, in the three-dimensional cut pattern, cut patterns by one or more cut surfaces positioned in relation to a geometric reference, wherein a cut surface in each case is defined by at least one parameter from the following list: cut line, cut line trajectory, cut direction, two-dimensional element class, two-dimensional element dimensions, two-dimensional element curvature and two-dimensional element orientation, and wherein an application sequence is assigned to the cut surfaces.

14. System (1) according to one of Claims 1 to 13, wherein the cut pattern generator (117) is configured to define the cut surfaces in the three-dimensional cut pattern in the form of macro-instructions.

15. System (1) according to one of Claims 1 to 14, wherein the cut surface editor (114) is configured to adapt, on the basis of user instructions, the at least one cut surface by at least one operation from the following list: scaling the size of the cut surface, delimiting the cut surface along one or more boundary lines, modifying a curvature of the cut surface, changing an orientation of the cut surface, changing a cut direction of the cut surface and changing a cut class of the cut surface, wherein the cut class is defined by at least one element from the following list of cut classes: cuts with a cut surface defined as a surface of the second order, cuts with a cut surface defined as a spline surface, cuts with a cut surface defined by a cut line and a cut direction and cuts with a cut surface defined by a stored three-dimensional cut pattern.

## Revendications

1. Système d'ordinateur (1) destiné à générer un motif de découpe tridimensionnel, qui définit une ou plusieurs découpes de tissu devant être réalisées dans un oeil humain (2) au moyen d'impulsions laser femtoseconde, comprenant :
une mémoire de données (18) contenant des données d'oeil (181) définissant un modèle d'oeil tridimensionnel ;
un générateur de référence (113) qui est conçu pour définir et stocker au moins une référence géométrique par rapport au motif d'oeil tridimensionnel ;
un éditeur de surface de découpe (114) qui est conçu pour définir une pluralité de surfaces de découpe sur la base d'instructions de l'utilisateur, et pour positionner la pluralité de surfaces de découpe par rapport à au moins une référence géométrique dans le modèle d'oeil tridimensionnel ; et
un générateur de motif de découpe (117) qui est conçu pour générer des motifs de découpe tridimensionnels pour la définition de découpes de tissu sur la base de la pluralité de surfaces de découpe déterminées et positionnées par l'utilisateur dans le modèle d'oeil et tridimensionnel et pour les stocker sous la forme de données de motifs de découpe (186) avec des macro-instructions ou des instructions de trames impulsionnelles pour commander un dispositif à laser ophtalmologique (3).

2. Système (1) selon la revendication 1, dans lequel l'éditeur de surface de découpe (114) est conçu pour définir l'au moins une surface de découpe sur la base d'une ligne de découpe définie par l'utilisateur et d'une trajectoire de ligne de découpe définie par l'utilisateur.

3. Système (1) selon l'une quelconque des revendications 1 ou 2, dans lequel l'éditeur de surface de découpe (114) est conçu pour définir l'au moins une surface de découpe sur la base d'au moins un élément de surface qui peut être sélectionné sur la base d'instructions de l'utilisateur dans une liste d'une pluralité d'éléments de surface différents.

4. Système (1) selon l'une quelconque des revendications 1 à 3, comprenant un sélecteur d'applicateur (112) qui est conçu pour déterminer, sur la base d'instructions de l'utilisateur, un élément d'application (34) devant être placé sur l'oeil (2) lors de la réalisation de la découpe de tissu ; et un module de déformation (12) qui est conçu pour déterminer, sur la base des données d'oeil (181) et de l'élément d'application déterminé (34), un modèle d'oeil tridimensionnel déformé qui représente l'oeil (2) dans l'état où l'élément d'application (34) est appliqué.

5. Système (1) selon la revendication 4, dans lequel le générateur de référence (113) est conçu pour définir la référence géométrique par rapport au modèle d'oeil tridimensionnel déformé et pour la stocker ; en ce que l'éditeur de surface de découpe (114) est conçu pour positionner l'au moins une surface de découpe par rapport à l'au moins une référence géométrique dans le modèle d'oeil tridimensionnel déformé ; et en ce que le générateur de motif de découpe (117) est conçu pour générer le motif de découpe tridimensionnel sur la base de l'au moins une surface de découpe ou de la pluralité de surfaces de découpe positionnée(s) dans le modèle d'oeil tridimensionnel déformé et pour le stocker.

6. Système (1) selon l'une quelconque des revendications 4 ou 5, dans lequel le module de déformation (12) est conçu pour déterminer, sur la base du motif de découpe tridimensionnel et de l'élément d'application déterminé (34), un motif de découpe tridimensionnel déformé basé sur un état déformé de l'au moins une surface de découpe ou de la pluralité de surfaces de découpe positionnée(s) dans le modèle d'oeil tridimensionnel, dans lequel l'état déformé est défini par le modèle d'oeil tridimensionnel déformé.

7. Système (1) selon l'une quelconque des revendications 1 à 6, comprenant un générateur de séquence (116) qui est conçu pour déterminer, pour des surfaces de découpe de tissu définies dans le motif de découpe tridimensionnel, une séquence d'exécution de manière automatique ou sur la base d'instructions de l'utilisateur, dans lequel il est fait en sorte d'éviter qu'une surface de découpe devant être générée préalablement conformément à la séquence d'exécution masque ou dégrade une irradiation focalisée de l'impulsion laser femtoseconde afin de générer une surface de découpe devant être générée ultérieurement conformément à la séquence d'exécution.

8. Système (1) selon la revendication 7, dans lequel le générateur de séquence (116) est conçu pour déterminer un masquage ou une dégradation d'une surface de découpe devant être générée ultérieurement par une surface de découpe devant être générée antérieurement sur la base d'un cône de rayonnement (32) défini par l'irradiation focalisée de l'impulsion laser femtoseconde.

9. Système (1) selon l'une quelconque des revendications 7 ou 8, dans lequel le générateur de séquence (116) est conçu pour déterminer la séquence d'exécution destinée aux surfaces de découpe de manière à ce qu'une charge thermique attendue de l'oeil (2) ne dépasse pas une valeur limite définie.

10. Système (1) selon l'une quelconque des revendications 1 à 9, comprenant un sélecteur de masque (111) qui est conçu pour déterminer, sur la base d'instructions de l'utilisateur, un masque de projection (33) devant être positionné devant l'oeil (2) lors de l'exécution de la découpe de tissu, et pour vérifier, sur la base du masque de projection (33) déterminé et d'un cône de rayonnement (32) défini par l'irradiation focalisée de l'impulsion laser femtoseconde, si les surfaces de découpe des découpes de tissu définies dans le motif de découpe tridimensionnel ne sont pas dégradées par le masque de projection (33).

11. Système (1) selon l'une quelconque des revendications 1 à 10, comprenant un module de visualisation (13) qui est conçu pour représenter, sur la base des données d'oeil (181), une visualisation de l'oeil (2) et un cône de rayonnement (32) défini par l'irradiation focalisée de l'impulsion laser femtoseconde sur un dispositif d'affichage (17) ; et un enregistreur de découpe (115) qui est conçu pour générer, sur la base d'instructions de l'utilisateur, dans l'oeil visualisé (2), par des mouvements du cône de rayonnement (32), une découpe de tissu virtuelle et pour stocker la découpe de tissu virtuelle sous la forme d'un motif de découpe tridimensionnel.

12. Système (1) selon l'une quelconque des revendications 1 à 11, comprenant un simulateur de découpe (14) qui est conçu pour simuler, sur la base des données d'oeil (181) et du motif de découpe tridimensionnel stocké, une exécution des découpes de tissu définies par le motif de découpe tridimensionnel et pour les visualiser sur un dispositif d'affichage (17) ; et un éditeur de motif de découpe (11) qui est conçu pour adapter le motif de découpe tridimensionnel stocké sur la base d'instructions de l'utilisateur par au moins une opération choisie dans la liste suivante : repositionnement d'une surface de découpe, réorientation d'une surface de découpe, modification d'une direction de découpe d'une surface de découpe, suppression d'une surface de découpe, suppression d'une partie de surface de découpe, modification d'une surface de découpe, ajout d'une surface de découpe, duplication d'une surface de découpe, modification d'une référence géométrique, modification d'une séquence d'exécution destinée aux surfaces de découpe, modification d'un élément d'application (34) devant être placé sur l'oeil (2) lors de l'exécution de la découpe de tissu, modification d'un masque de projection (33) devant être positionné devant l'oeil (2) lors de l'exécution de la découpe de tissu, et modification d'un cône de rayonnement (32) défini par l'irradiation focalisée de l'impulsion laser femtoseconde.

13. Système (1) selon l'une quelconque des revendications 1 à 12, dans lequel le générateur de motif de découpe (117) est conçu pour définir des découpes de tissu dans le motif de découpe tridimensionnel au moyen d'une surface de découpe ou d'une pluralité de surfaces de découpe positionnée(s) par rapport à une référence géométrique, dans lequel une surface de découpe respective est définie par au moins un paramètre sélectionné dans la liste suivante : une ligne de découpe, une trajectoire de ligne de découpe, une direction de découpe, une classe d'élément de surface, des mesures d'élément de surface, une courbure d'élément de surface, et une orientation d'élément de surface, et dans lequel une séquence d'exécution est associée aux surfaces de découpe.

14. Système (1) selon l'une quelconque des revendications 1 à 13, dans lequel le générateur de motif de découpe (117) est conçu pour définir les surfaces de découpe dans le motif de découpe tridimensionnel sous forme de macro-instructions.

15. Système (1) selon l'une quelconque des revendications 1 à 14, dans lequel l'éditeur de surface de découpe (114) est conçu pour adapter l'au moins une surface de découpe sur la base d'instructions de l'utilisateur au moyen d'au moins une opération sélectionnée dans la liste suivante : modification de l'échelle de la taille de la surface de découpe, limitation de la surface de découpe le long d'une ou plusieurs lignes de délimitation, modification d'une courbure de la surface de découpe, modification d'une orientation de la surface de découpe, modification d'une direction de découpe de la surface de découpe et modification d'une classe de découpe de la surface de découpe, dans lequel la classe de découpe est définie par au moins un élément sélectionné dans la liste de classes de découpe suivante : une découpe ayant une surface de découpe définie sous la forme d'une surface de deuxième ordre, une découpe ayant une surface de découpe définie sous la forme d'une surface de type spline, une découpe ayant une surface de découpe définie par une ligne de découpe et une direction de découpe, et une découpe ayant une surface de découpe définie par un motif de découpe tridimensionnel stocké.
